# EUROPÄISCHE PATENTSCHRIFT

(11) **EP 2 848 223 B1**
(45) Veröffentlichungstag und Bekanntmachung des Hinweises auf die Patenterteilung: **03.04.2019**
(21) Anmeldenummer: 14184705.3
(22) Anmeldetag: 15.09.2014
(51) Int. Cl.: A61B 17/70

(54) **VERBINDUNGSELEMENT UND WIRBELSÄULENSTABILISIERUNGSSYSTEM**
Connecting element and spinal column stabilzation system
Élément de liaison et système de stabilisation de la colonne vertébrale

(30) Priorität: 16.09.2013 DE 102013110173
(43) Veröffentlichungstag der Anmeldung: 18.03.2015
(73) Patentinhaber: AESCULAP AG, 78532 Tuttlingen (DE)
(72) Erfinder: Beger, Jens, 78532 Tuttlingen (DE); Krüger, Sven, 78647 Trossingen (DE); Celmerowski, Beate, 78594 Gunningen (DE)
(74) Vertreter: Hoeger, Stellrecht & Partner Patentanwälte mbB

(56) Entgegenhaltungen:
- DE-U1- 9 402 695
- FR-A1- 2 846 222
- US-A1- 2006 064 090
- US-A1- 2011 009 906

## Beschreibung

Die vorliegende Erfindung betrifft Verbindungselement für ein Wirbelsäulenstabilisierungssystem mit einem ersten Ende zum Festlegen an einer ersten Knochenbefestigungseinrichtung, einem zweiten Ende zum Festlegen an einer zweiten Knochenbefestigungseinrichtung und einem zwischen den beiden Enden angeordneten oder ausgebildeten, eine Längsachse definierenden Zwischenabschnitt, wobei mindestens eines der beiden Enden in Form eines Kopplungselements ausgebildet ist zum Festlegen des Verbindungselements an einer Knochenbefestigungseinrichtung in mindestens einer definierten Orientierung bezogen auf die Längsachse und wobei das Kopplungselement eine von einem Kreiszylinder abweichende Form und eine Verbindungselementanlagefläche aufweist zum Anlegen an ein weiteres Verbindungselement, insbesondere an ein Ende des weiteren Verbindungselements, wobei die Verbindungselementanlagefläche mindestens teilweise in Form einer hohlzylindrischen Wandfläche ausgebildet ist zum Aufnehmen eines in Form eines Kreiszylinders ausgebildetes und sich in Richtung der Längsachse oder parallel zu dieser erstreckendes Endes eines weiteren Verbindungselements, wobei das Kopplungselement einen Kopplungselementgrundkörper und mindestens ein am Kopplungselementgrundkörper angeordnetes oder ausgebildetes Kopplungsglied umfasst, wobei sich das mindestens eine Kopplungsglied in einer Kopplungsgliedrichtung erstreckt, welche quer zur Längsachse verläuft, wobei das mindestens eine Kopplungsglied in Form eines Kopplungsvorsprungs ausgebildet ist.

Ferner betrifft die vorliegende Erfindung ein Wirbelsäulenstabilisierungssystem umfassend mindestens eine erste Knochenbefestigungseinrichtung, mindestens eine zweite Knochenbefestigungseinrichtung und mindestens ein Verbindungselement, welches mindestens eine Verbindungselement ein erstes Ende zum Festlegen an der mindestens einen ersten Knochenbefestigungseinrichtung, ein zweites Ende zum Festlegen an der mindestens zweiten Knochenbefestigungseinrichtung und einen zwischen den beiden Enden angeordneten oder ausgebildeten, eine Längsachse definierenden Zwischenabschnitt umfasst.

Verbindungselemente und Wirbelsäulenstabilisierungssysteme der eingangs beschriebenen Art sind beispielsweise aus der EP 1 658 815 B1 bekannt. Die in dieser europäischen Patentschrift beschriebenen Verbindungselemente umfassen elastische Elemente und weisen erste und zweite Enden auf, die jeweils kreiszylindrisch ausgebildet sind und deren Längsachsen zusammen fallen. Sie können in entsprechenden Aufnahmen von Knochenbefestigungseinrichtungen klemmend festgelegt werden.

Nachteilig bei den bekannten Verbindungselementen ist jedoch, dass der Zwischenabschnitt nur mit sehr großem Aufwand definiert ausrichtbar ist.

Weitere Wirbelsäulenstabilisierungssysteme sind aus der DE 94 02 695 U1, der US 2011/0009906 A1, der US 2006/0064090, der FR 2 846 222 und der DE 20 2010 008 865 U1 bekannt.

Es ist daher eine Aufgabe der vorliegenden Erfindung, ein Verbindungselement und ein Wirbelsäulenstabilisierungselement der eingangs beschriebenen Art so zu verbessern, dass deren Handhabbarkeit vereinfacht wird.

Diese Aufgabe wird bei einem Verbindungselement der eingangs beschriebenen Art erfindungsgemäß dadurch gelöst, dass der Kopplungsvorsprung ballig, kugelig, zylindrisch oder in Form einer Kombination einer oder mehrerer dieser Formen ausgebildet ist und dass der Kopplungselementgrundkörper drei oder mehr Kopplungsglieder umfasst.

Durch diese besondere Ausgestaltung wird es auf einfache und sichere Weise für einen Operateur möglich, das Verbindungselement definiert orientiert zu implantieren, das heißt insbesondere definiert orientiert an den Knochenbefestigungseinrichtungen festzulegen. In Form von Kreiszylindern ausgebildete Enden, wie sie bei den aus der EP 1 658 815 B1 bekannten Verbindungselementen vorgesehen sind, definieren aufgrund ihrer rotationssymmetrischen Form gerade keine Vorzugsrichtung. Sie ermöglichen zwar eine beliebige Orientierung des Verbindungselements und damit Ausrichtung des Zwischenabschnitts bezogen auf seine Längsachse, doch kann gerade dies nachteilig sein, wenn beispielsweise der Zwischenabschnitt ebenfalls eine Vorzugsrichtung definiert, was insbesondere bei flexiblen oder teilweise flexiblen Zwischenabschnitten möglich ist, und daher in definierter Weise zu den Knochenbefestigungseinrichtungen ausgerichtet werden soll. Genau dieses Problem wird durch die besondere Ausbildung mindestens einer der beiden Enden, es können auch beide Enden so ausgebildet sein, in Form eines Kopplungselements, das eine von einem Kreiszylinder abweichende Form aufweist. Insbesondere ist das Kopplungselement definitionsgemäß, anders als ein Kreiszylinder, nicht vollständig rotationssymmetrisch ausgebildet, so dass selbst bei einer Verbindungselementaufnahme, die an einer Knochenbefestigungseinrichtung vorgesehen ist und die rotationssymmetrisch oder im Wesentlichen rotationssymmetrisch ausgebildet sein kann, eine definierte Orientierung des Verbindungselements und damit von dessen Zwischenabschnitt bezogen auf die Längsachse möglich ist, und zwar ohne das ein Operateur besondere Anstrengungen unternehmen muss. Vorzugsweise ist genau eine definierte Orientierung aufgrund einer Formgebung des Kopplungselements möglich. Damit das Verbindungselement auf einfache und sichere Weise mit einer definierten Orientierung relativ zu mindestens einer Knochenbefestigungseinrichtung an dieser festgelegt werden kann, ist es günstig, dass das Kopplungselement einen Kopplungselementgrundkörper und mindestens ein am Kopplungselementgrundkörper angeordnetes oder ausgebildetes Kopplungsglied umfasst. Das mindestens eine Kopplungsglied kann insbesondere dazu dienen, mit einem entsprechenden Teil an der Knochenbefestigungseinrichtung zusammenzuwirken oder auch mit einem weiteren Kopplungsglied an einem weiteren Verbindungselement, beispielsweise um so das Verbindungselement bei Bedarf verlängern zu können. Insbesondere kann das mindestens eine Kopplungsglied ausgebildet sein zum formschlüssigen in Eingriff bringen mit entsprechend vorgesehenen weiteren Kopplungsgliedern an anderen Verbindungselementen oder an einer Knochenbefestigungseinrichtung. Das mindestens eine Kopplungsglied erstreckt sich in einer Kopplungsgliedrichtung, welche parallel oder quer zur Längsachse verläuft. Je nachdem, wie insbesondere eine Verbindungselementaufnahme an einer Knochenbefestigungseinrichtung ausgebildet ist, kann durch entsprechende Vorgabe der Kopplungsgliedrichtung eine optimale Verbindung mit einer Knochenbefestigungseinrichtung erreicht werden, insbesondere kann so eine Verdrehung oder eine axiale Verschiebung relativ zur Knochenbefestigungseinrichtung einfach und sicher unterbunden werden. Vorteilhafterweise ist das mindestens eine Kopplungsglied in Form eines Kopplungsvorsprungs ausgebildet. So kann es mit entsprechenden Ausnehmungen in gewünschter Weise zusammenwirken. Gemäß der Erfindung ist vorgesehen, dass das Kopplungselement eine Verbindungselementanlagefläche aufweist zum Anlegen an ein weiteres Verbindungselement. Insbesondere kann die Verbindungselementanlagefläche ausgebildet sein zum Anlegen an ein Ende des weiteren Verbindungselements. Eine solche Ausgestaltung ermöglicht eine besonders gute und sichere Kopplung mit einem weiteren Verbindungselement. Die Verbindungselementanlagefläche ist mindestens teilweise in Form einer hohlzylindrischen Wandfläche ausgebildet. Eine solche Ausgestaltung ermöglicht es insbesondere, ein in Form eines Kreiszylinders ausgebildetes und sich in Richtung der Längsachse oder parallel zu dieser erstreckendes Ende eines weiteren Verbindungselements aufzunehmen. Der Kopplungsvorsprung ist ballig, kugelig, zylindrisch, konisch, quaderförmig oder in Form einer Kombination einer oder mehrerer dieser Formen ausgebildet. Insbesondere kann der Kopplungsvorsprung an eine Verbindungselementaufnahme einer Knochenbefestigungseinrichtung angepasst sein, um so insbesondere ein formschlüssiges in Eingriff bringen zu ermöglichen. Günstigerweise weist das Verbindungselement zwei, drei oder mehr Kopplungsglieder auf. So können es insbesondere auch vier, fünf oder sechs Kopplungsglieder sein. Insbesondere können die Kopplungsglieder in unterschiedliche Richtungen weisende Kopplungsgliedlängsachsen aufweisen, um so eine Bewegung des Verbindungselements relativ zu einer Knochenbefestigungseinrichtung oder einem weiteren Verbindungselement in unterschiedlichen Richtungen zu beschränken.

Günstigerweise sind beide Enden in Form eines Kopplungselements ausgebildet, welches eine von einem Kreiszylinder abweichende Form aufweist. So kann eine optimale Verbindung mit zwei Knochenbefestigungseinrichtungen erreicht werden.

Das Verbindungselement kann auf einfache Weise hergestellt werden, wenn das Kopplungselement einstückig ausgebildet ist. Beispielsweise kann es durch spanende Bearbeitung ausgebildet oder durch Gießen hergestellt werden.

Um eine Stabilität des Verbindungselements zu verbessern, ist es vorteilhaft wenn es einstückig ausgebildet ist.

Auf einfache Weise konstruieren lässt sich das Verbindungselement, wenn der Kopplungselementgrundkörper in Form eines geraden oder schiefen allgemeinen Zylinders ausgebildet ist und wenn eine vom Zylinder definierte Zylinderlängsachse quer zur Längsachse verläuft. Ein allgemeiner Zylinder weist in zueinander parallelen Schnittebenen dieselbe Querschnittsfläche auf. Eine Form der Querschnittsfläche kann beliebig sein, weicht jedoch erfindungsgemäß von einem Kreis ab, um eine undefinierte Ausrichtung des Zwischenabschnitts relativ zu den Knochenbefestigungseinrichtungen zu vermeiden. Alternativ kann eine Zylinderlängsachse des allgemeinen Zylinders auch parallel zur Längsachse des Verbindungselements verlaufen.

Günstig ist es, wenn der Kopplungselementgrundkörper eine Querschnittsfläche aufweist, die kreisförmig, viereckig oder in Form eines Vielrunds ausgebildet ist. Insbesondere kann die Querschnittsfläche rechteckig oder quadratisch ausgebildet sein. Derartige Grundformen lassen sich einfach herstellen und ermöglichen bereits eine definierte Ausrichtung des Verbindungselements relativ zu einer Knochenbefestigungseinrichtung.

Gemäß einer weiteren bevorzugten Ausführungsform der Erfindung kann vorgesehen sein, dass die Kopplungsaufnahme hohlballig, hohlkugelig, hohlzylindrisch, quaderförmig, in Form einer Durchbrechung des Kopplungselementgrundkörpers oder in Form einer Kombination einer oder mehrerer dieser Formen ausgebildet ist. Insbesondere kann eine derart geformte Kopplungsaufnahme mit einem entsprechend geformten Kopplungsvorsprung eines weiteren Verbindungselements zusammenwirken, um so beispielsweise zwei Enden von zwei Verbindungselementen miteinander zu koppeln und gemeinsam an einer Knochenbefestigungseinrichtung festzulegen.

Günstigerweise kann die Kopplungsaufnahme in Form eines Rücksprungs oder einer Nut ausgebildet sein. Diese können sich parallel oder quer zur Längsachse erstrecken.

Vorteilhaft ist es, wenn die Kopplungsaufnahme in einer Richtung quer und/oder parallel zur Längsachse geöffnet ist. So kann sie im Zusammenwirken mit einem weiteren Kopplungsglied eine Relativbewegung des Verbindungselements zu einer Knochenbefestigungseinrichtung parallel und/quer zur Längsachse verhindern.

Auf einfache Weise lässt sich das Verbindungselement konstruieren, wenn das Kopplungselement zwei bezogen auf eine die Längsachse enthaltende Spiegelebene spiegelsymmetrisch angeordnete oder ausgebildete Kopplungsglieder aufweist. Beispielsweise können dies parallel zueinander verlaufende Rücksprünge oder Nuten sein.

Ferner kann es günstig sein, wenn das Kopplungselement bezogen auf eine die Längsachse enthaltende Spiegelebene spiegelsymmetrisch ausgebildet ist. So können je nach Form des Kopplungselements beispielsweise eine, zwei oder vier definierte Orientierungen des Verbindungselements relativ zu einer Knochenbefestigungseinrichtung vorgegeben werden.

Besonders einfach ausbilden lässt sich das Verbindungselement, wenn es bezogen auf eine die Längsachse enthaltende Spiegelebene spiegelsymmetrisch ausgebildet ist.

Um das Verbindungselement besonders platzsparend und gegebenenfalls mit einem weiteren Verbindungselement gemeinsam in einer Verbindungselementaufnahme einer Befestigungseinrichtung festzulegen, ist es vorteilhaft, wenn ein Schwerpunkt des Kopplungselements von der Längsachse beabstandet ist.

Das Einführen des Verbindungselements in den Körper eines Patienten, insbesondere bei minimalinvasiven Eingriffen, lässt sich verbessern und eine Verletzungsgefahr minimieren, wenn eine vom Zwischenabschnitt weg weisende Endfläche des Kopplungselements bezogen auf die Längsachse geneigt oder abgerundet ist.

Auf einfache Weise herstellen lässt sich eine solche Endfläche, wenn eine von der Endfläche definierte Endflächenebene und die Längsachse einen Endflächenneigungswinkel einschließen, welcher in einem Bereich von etwa 30° bis 90° liegt, vorzugsweise in einem Bereich von etwa 45° bis 75°.

Vorteilhaft ist es, wenn der Kopplungselementgrundkörper einen Ausschnitt eines Kreiszylinders umfasst, welcher eine sich quer zur Längsachse verlaufende Kreiszylinderlängsachse aufweist. Beispielsweise kann der Kopplungselementgrundkörper so in eine korrespondierende Ausnehmung eingreifen, so dass eine Bewegung parallel zur Längsachse des Verbindungselements relativ zu einer Knochenbefestigungseinrichtung oder einem weiteren Verbindungselement einfach und sicher beschränkt werden kann.

Ferner kann es günstig sein, wenn der Kopplungselementgrundkörper einen ebenen Flächenabschnitt aufweist, welcher eine Ebene definiert, die parallel oder quer zur Längsachse verläuft. Insbesondere ein Flächenabschnitt, welcher eine parallel zur Längsachse verlaufende Ebene definiert, ermöglicht eine flächige Anlage beispielsweise mit einem Fixierelement zum Festlegen des Verbindungselements an der Knochenbefestigungseinrichtung. Ferner können so auch zwei Enden von zwei Verbindungselementen flächig aneinander anliegen.

Günstig ist es, wenn die Verbindungselementanlagefläche mindestens teilweise in Form eines ebenen Anlageflächenabschnitts ausgebildet ist. Eine solche Verbindungselementanlagefläche lässt sich einfach herstellen und an eine korrespondierende ebene Verbindungselementanlagefläche anlegen.

Gemäß einer weiteren bevorzugten Ausführungsform der Erfindung kann vorgesehen sein, dass der Kopplungselementgrundkörper zwei, drei oder mehr relativ zueinander geneigte, ebene oder vom Kopplungselementgrundkörper weg weisend konkav gekrümmte Kopplungselementgrundkörperseitenflächen aufweist. Insbesondere können zueinander geneigte Kopplungselementgrundkörperseitenflächen, auch wenn sie eben sind, eine insgesamt vom Kopplungselementgrundkörper weg weisend konkave Form ausbilden. Ein solcher Kopplungselementgrundkörper kann insbesondere mit einer korrespondierenden konvexen Seitenfläche einer Knochenbefestigungseinrichtung zusammenwirken.

Die Herstellung des Verbindungselements lässt sich weiter vereinfachen, wenn aneinander angrenzende ebene Kopplungselementgrundkörperseitenflächen zwischen sich einen stumpfen Winkel einschließen.

Günstig ist es, wenn der der Zwischenabschnitt in Form eines streifenförmigen, gewundenen Blattfederelements ausgebildet ist und mindestens eine in einer Ausnehmungsrichtung quer zu einer vom Zwischenabschnitt definierten Längsachse seitlich geöffnete Ausnehmung aufweist. Ein solcher Zwischenabschnitt ermöglicht die Vorgabe einer definierten Elastizität des Verbindungselements sowie einer definierten Anisotropie der Elastizität in Umfangsrichtung bezogen auf die Längsachse. Ferner lässt sich das Verbindungselement mit den Ausnehmungen einfach und sicher handhaben, beispielsweise mit einem korrespondierend geformten Halteinstrument. Ferner können in die Ausnehmungen auch temporär Blockierelemente eingesetzt werden, um die Elastizität des Verbindungselements, insbesondere bei der Implantation, temporär ganz oder teilweise einzuschränken.

Gemäß einer weiteren bevorzugten Ausführungsform der Erfindung kann vorgesehen sein, dass das Kopplungselement mindestens eine Vorzugsrichtung quer zur Längsachse definiert und dass die Vorzugsrichtung und die Ausnehmungsrichtung einen Orientierungswinkel einschließen, welcher in einem Bereich von 0° bis 180° liegt. Vorzugsweise liegt der Orientierungswinkel in einem Bereich von 0° bis 90°. Durch diese Ausgestaltung des Kopplungselements kann eine Orientierung des Verbindungselements bezogen auf die Längsachse vorgegeben werden und so auch eine Orientierung der Ausnehmungsrichtung beispielsweise relativ zu dem Teil der Knochenbefestigungseinrichtung, an dem die Verbindungselementaufnahme zum Aufnehmen eines Endes des Verbindungselements ausgebildet ist.

Vorzugsweise definiert das Kopplungselement zwei oder mehr Vorzugsrichtungen. Dies ermöglicht es einem Operateur, das Verbindungselement in unterschiedlichen, jedoch definierten Orientierungen relativ zur Knochenbefestigungseinrichtung definiert anzuordnen.

Vorteilhaft ist es, wenn ein Vorzugswinkel zwischen zwei Vorzugsrichtungen des Kopplungselements einem Winkel 180°/n entspricht, wobei n die Zahl der vom Kopplungsglied insgesamt definierten Vorzugsrichtungen ist. Definiert beispielsweise das Kopplungselement zwei Vorzugsrichtungen, so beträgt der Vorzugswinkel insbesondere 90°.

Vorteilhafterweise ist das Verbindungselement aus einem metallischen Material oder aus einem Kunststoff hergestellt. Vorzugsweise sind die Materialien körperverträglich und weisen die für die Anwendung erforderliche Stabilität auf.

Günstig ist es, wenn das metallische Material Titan, eine Titanlegierung oder eine Kobalt-Chrom-Legierung ist oder enthält. Derartige Materialien sind als Implantatmaterialien hervorragend geeignet.

Ferner ist es vorteilhaft, wenn der Kunststoff Polyetheretherketon oder Kohlefaser verstärktes Polyetheretherketon ist oder enthält. Ein solches Kunststoffmaterial ist ebenfalls biokompatibel und weist die für die Anwendung erforderliche Stabilität auf.

Die eingangs gestellte Aufgabe wird bei einem Wirbelsäulenstabilisierungssystem der eingangs beschriebenen Art erfindungsgemäß dadurch gelöst, dass das Verbindungselement in Form eines der oben beschriebenen Verbindungselemente ausgebildet ist.

Wirbelsäulenstabilisierungssysteme mit den oben beschriebenen, erfindungsgemäßen Verbindungselementen weisen dann ebenfalls die dort dargelegten Vorteile auf.

Günstig ist es, wenn die mindestens eine erste und/oder die mindestens zweite Knochenbefestigungseinrichtung in Form von Pedikelschrauben ausgebildet sind. Insbesondere können die Pedikelschrauben in Form polyaxialer Pedikelschrauben ausgebildet sein. Pedikelschrauben sind insbesondere ausgebildet, um an Pedikeln von Wirbeln festgelegt zu werden. Werden Verbindungselemente an den Pedikelschrauben befestigt, kann so eine Beweglichkeit einer Wirbelsäule eines Patienten in definierter Weise ganz oder teilweise eingeschränkt werden.

Ferner ist es günstig, wenn die mindestens eine erste und/oder die mindestens eine zweite Knochenbefestigungseinrichtung einen Befestigungsteil und einen Halteteil umfassen und wenn der Halteteil eine Verbindungselementaufnahme zum Aufnehmen mindestens eines Endes eines Verbindungselements aufweist. Das Halteteil kann optional beweglich oder unbeweglich mit dem Befestigungsteil verbunden sein. Vorzugsweise ist der Halteteil in einer Implantationsstellung relativ zum Befestigungsteil festlegbar. Dies gestattet es, den Halteteil relativ zum Befestigungsteil zunächst in gewünschter Weise auszurichten und dann Festzulegen, beispielsweise mit gleichzeitiger Festlegung des Verbindungselements in der Verbindungselementaufnahme.

Auf besonders einfache Weise lässt sich eine Verbindungselementaufnahme ausbilden, wenn der Halteteil zwei die Verbindungselementaufnahme seitlich begrenzende, symmetrisch zueinander angeordnete Wandabschnitte aufweist. Insgesamt lässt sich so ein Halteteil ausbilden, welches eine Gabelform aufweist. Man kann auch sagen, die Verbindungselementaufnahme wird ausgebildet durch einen Schlitz des Halteteils.

Günstig ist es, wenn die mindestens eine erste und/oder die mindestens eine zweite Knochenbefestigungseinrichtung eine Fixierschraube mit einem Außengewinde umfasst und wenn die Verbindungselementaufnahme ein zum Außengewinde korrespondierendes Innengewinde aufweist. Die Fixierschraube gestattet es auf einfache Weise, ein in die Verbindungselementaufnahme eingelegtes Ende eines Verbindungselements temporär oder dauerhaft an der ersten oder zweiten Knochenbefestigungseinrichtung zu fixieren.

Ferner ist es vorteilhaft, wenn der Kopplungselementgrundkörper einen ebenen Klemmflächenabschnitt für ein distales Ende der Fixierschraube aufweist, welcher Klemmflächenabschnitt eine Ebene definiert, die parallel oder im Wesentlichen parallel zur Längsachse verläuft. So ist es insbesondere möglich, mit einem distalen Ende der Fixierschraube flächig den Klemmflächenabschnitt zu klemmen.

Vorzugsweise ist der Zwischenabschnitt mindestens teilweise flexibel ausgebildet. Mit einem solchen Zwischenabschnitt kann eine definierte Beweglichkeit des Wirbelsäulenstabilisierungssystems vorgegeben werden. Günstig ist es, wenn der Zwischenabschnitt eine anisotrope Flexibilität vorgibt. Beispielsweise kann so eine Flexion und Extension in der Medianebene eines Patienten teilweise ermöglicht werden, ein seitliches Abwinkeln der Wirbelsäule dagegen nicht.

Gemäß einer weiteren bevorzugten Ausführungsform der Erfindung kann vorgesehen sein, dass das Wirbelsäulenstabilisierungssystem zwei Verbindungselemente umfasst, die zueinander korrespondierende Kopplungselemente aufweisen, welch egemeinsam in einer Verbindungselementaufnahme der mindestens einen ersten und/oder der mindestens einen zweiten Knochenbefestigungseinrichtung festlegbar sind. So kann ein besonders kompakter Aufbau des Wirbelsäulenstabilisierungssystems erreicht werden. Insbesondere können Verbindungselemente so auf einfache Weise verlängert werden.

Insbesondere können auch nur kurze Verbindungselemente genutzt werden, die eine Länge aufweisen, die gerade die Verbindung zwischen zwei Knochenbefestigungseinrichtungen ermöglicht. Außerdem kann beispielsweise mit einer einzigen Fixierschraube nicht nur ein Verbindungselement klemmend an der Knochenbefestigungseinrichtung festgelegt werden, sondern zwei Verbindungselemente an einer einzigen Knochenbefestigungseinrichtung.

Vorteilhaft ist es, wenn die Kopplungselemente der beiden Verbindungselemente zueinander korrespondierende Kopplungsglieder aufweisen, die in einer Kopplungsstellung, in welcher die beiden Kopplungselemente gemeinsam in einer Verbindungselementaufnahme der mindestens einen ersten und/oder der mindestens einen zweiten Knochenbefestigungseinrichtung gehalten sind, kraft- und/oder formschlüssig zusammenwirken oder miteinander in Eingriff stehen. Die Kopplungsglieder ermöglichen eine direkte, insbesondere formschlüssige Kopplung zwischen den beiden Verbindungselementen, und nicht nur zwischen den Verbindungselementen und der Knochenbefestigungseinrichtung.

Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische Gesamtansicht eines an einer Wirbelsäule festgelegten Wirbelsäulenstabilisierungssystems;
- Figur 2:: eine Ansicht eines an zwei Knochenschrauben festgelegten ersten, nicht erfindungsgemäßen Ausführungsbeispiels eines Verbindungselements;
- Figur 3:: eine Explosionsdarstellung der Anordnung aus Figur 2;
- Figur 4:: eine teilweise geschnittene Ansicht der Anordnung in Figur 2 bei längs Linie 4-4;
- Figur 5:: eine Ansicht in Richtung des Pfeils A in Figur 4;
- Figur 6:: eine Ansicht in Richtung des Pfeils B in Figur 5;
- Figur 7:: eine Seitenansicht des Verbindungselements aus Figur 3;
- Figur 8:: eine Ansicht des Verbindungselements aus Figur 7 in Richtung des Pfeils C;
- Figur 9:: eine Ansicht des Verbindungselements aus Figur 7 in Richtung des Pfeils D;
- Figur 10:: eine schematische perspektivische Ansicht eines zweiten, nicht erfindungsgemäßen Ausführungsbeispiels eines Verbindungselements beim Festlegen an zwei Knochenschrauben;
- Figur 11:: eine Ansicht der Anordnung aus Figur 10 in Richtung des Pfeils E;
- Figur 12:: eine Ansicht der Anordnung aus Figur 11 in Richtung des Pfeils F;
- Figur 13:: eine Seitenansicht des zweiten Ausführungsbeispiels eines Verbindungselements;
- Figur 14:: eine Ansicht des Verbindungselements aus Figur 13 in Richtung des Pfeils G;
- Figur 15:: eine Ansicht des Verbindungselements aus Figur 13 in Richtung des Pfeils H;
- Figur 16:: eine schematische perspektivische Ansicht eines dritten, nicht erfindungsgemäßen Ausführungsbeispiels eines Verbindungselements beim Festlegen an zwei Knochenschrauben;
- Figur 17:: eine Ansicht der Anordnung aus Figur 16 in Richtung des Pfeils I;
- Figur 18:: eine Seitenansicht des Verbindungselements aus Figur 17;
- Figur 19:: eine Ansicht des Verbindungselements aus Figur 18 in Richtung des Pfeils J;
- Figur 20:: eine schematische perspektivische Ansicht eines vierten Ausführungsbeispiels eines Verbindungselements beim Festlegen an einer Knochenschraube mit einem weiteren stabförmigen Verbindungselement;
- Figur 21:: eine Explosionsdarstellung der Anordnung aus Figur 20;
- Figur 22:: eine Ansicht der Anordnung aus Figur 21 in Richtung des Pfeils K;
- Figur 23:: eine Ansicht der beiden Verbindungselemente in Figur 22 in Richtung des Pfeils L;
- Figur 24:: eine Ansicht des Verbindungselements aus Figur 25 in Richtung des Pfeils M;
- Figur 25:: eine Seitenansicht des Verbindungselements aus Figur 23;
- Figur 26:: eine schematische perspektivische Ansicht eines fünften, nicht erfindungsgemäßen Ausführungsbeispiels eines Verbindungselements beim Festlegen an einer Knochenschraube;
- Figur 27:: eine Explosionsdarstellung der Anordnung aus Figur 26;
- Figur 28:: eine teilweise geschnittene Ansicht der Anordnung aus Figur 27 längs Linie 28-28;
- Figur 29:: eine Ansicht des Verbindungselements aus Figur 30 in Richtung des Pfeils N;
- Figur 30:: eine Seitenansicht des Verbindungselements aus Figur 27;
- Figur 31:: eine schematische perspektivische Ansicht eines sechsten, nicht erfindungsgemäßen Ausführungsbeispiels eines Verbindungselements beim Festlegen an einer Knochenschraube;
- Figur 32:: eine teilweise geschnittene Ansicht längs Linie 32-32 aus Figur 31;
- Figur 33:: eine Ansicht des Verbindungselements aus Figur 34 in Richtung des Pfeils O;
- Figur 34:: eine Seitenansicht des Verbindungselements aus Figur 32;
- Figur 35:: eine schematische perspektivische Ansicht eines siebten, nicht erfindungsgemäßen Ausführungsbeispiels eines Verbindungselements beim Festlegen an zwei Knochenschrauben;
- Figur 36:: eine teilweise geschnittene Ansicht längs Linie 36-36 in Figur 35;
- Figur 37:: eine Ansicht des Verbindungselements aus Figur 38 in Richtung des Pfeils P;
- Figur 38:: eine Seitenansicht des Verbindungselements aus Figur 35;
- Figur 39:: eine perspektivische Ansicht eines achten, nicht erfindungsgemäßen Ausführungsbeispiels eines Verbindungselements;
- Figur 40:: eine Ansicht des Verbindungselements aus Figur 39 in Richtung des Pfeils Q;
- Figur 41:: eine schematische perspektivische Ansicht eines neunten, nicht erfindungsgemäßen Ausführungsbeispiels eines Verbindungselements beim Festlegen an zwei Knochenschrauben;
- Figur 42:: eine Ansicht der Anordnung aus Figur 41 in Richtung des Pfeils R;
- Figur 43:: eine Ansicht des Verbindungselements aus Figur 44 in Richtung des Pfeils T;
- Figur 44:: eine Seitenansicht des Verbindungselements aus Figur 41;
- Figur 45:: eine Ansicht des Verbindungselements aus Figur 44 in Richtung des Pfeils S;
- Figur 46:: eine perspektivische Ansicht eines zehnten, nicht erfindungsgemäßen Ausführungsbeispiels eines Verbindungselements;
- Figur 47:: eine Ansicht des Verbindungselements aus Figur 46 in Richtung des Pfeils U;
- Figur 48:: eine Ansicht des Verbindungselements aus Figur 47 in Richtung des Pfeils V;
- Figur 49:: eine schematische perspektivische Ansicht eines elften, nicht erfindungsgemäßen, und eines zwölften, nicht erfindungsgemäßen Ausführungsbeispiels eines Verbindungselements beim gemeinsamen Festlegen an einer einzigen Knochenschraube;
- Figur 50:: eine Explosionsdarstellung der Anordnung aus Figur 49;
- Figur 51:: eine teilweise geschnittene Ansicht der Anordnung aus Figur 49 längs Linie 51-51;
- Figur 52:: eine Ansicht der beiden Verbindungselemente aus Figur 53 in Richtung des Pfeils W;
- Figur 53:: eine teilweise geschnittene Seitenansicht der miteinander gekoppelten Verbindungselemente aus Figur 49;
- Figur 54:: eine schematische perspektivische Ansicht eines dreizehnten, nicht erfindungsgemäßen, und eines vierzehnten, nicht erfindungsgemäßen Ausführungsbeispiels eines Verbindungselements beim gemeinsamen Festlegen einer einzigen Knochenschraube;
- Figur 55:: eine teilweise geschnittene Ansicht längs Linie 55-55 in Figur 54;
- Figur 56:: eine teilweise geschnittene Seitenansicht der beiden miteinander gekoppelten Verbindungselemente aus Figur 55;
- Figur 57:: eine schematische perspektivische Ansicht eines fünfzehnten, nicht erfindungsgemäßen, und eines sechzehnten, nicht erfindungsgemäßen Ausführungsbeispiels eines Verbindungselements beim gemeinsamen Festlegen an einer einzigen Knochenschraube;
- Figur 58:: eine teilweise geschnittene Ansicht der Anordnung aus Figur 57 längs Linie 58-58;
- Figur 59:: eine Explosionsdarstellung der Anordnung aus Figur 57;
- Figur 60:: eine Draufsicht auf die miteinander gekoppelten Verbindungselemente aus Figur 57;
- Figur 61:: eine Schnittansicht längs Linie 61-61 in Figur 60;
- Figur 62:: eine schematische perspektivische Ansicht eines siebzehnten Ausführungsbeispiels eines Verbindungselements, das mit einem weiteren stabförmigen Verbindungselement an einer Knochenschraube festgelegt ist;
- Figur 63:: eine teilweise geschnittene Ansicht der Anordnung aus Figur 62 längs Linie 63-63;
- Figur 64:: eine perspektivische Ansicht des Verbindungselements aus Figur 63 von oben;
- Figur 65:: eine perspektivische Ansicht des Verbindungselements aus Figur 64 von unten;
- Figur 66:: eine Ansicht des Verbindungselements aus Figur 67 in Richtung des Pfeils Y; und
- Figur 67:: eine Ansicht des Verbindungselements aus Figur 66 in Richtung des Pfeils X.

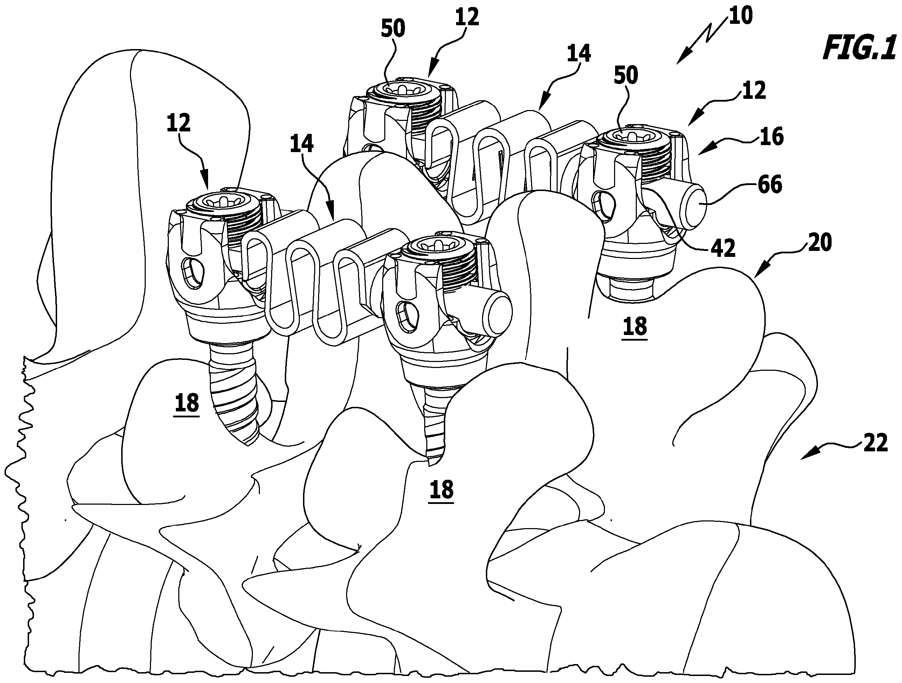

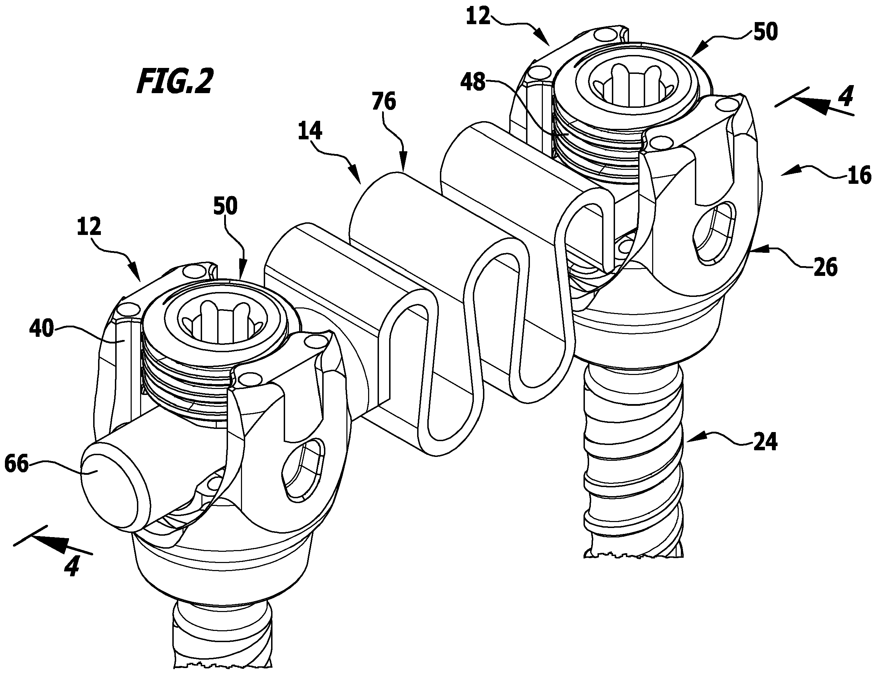

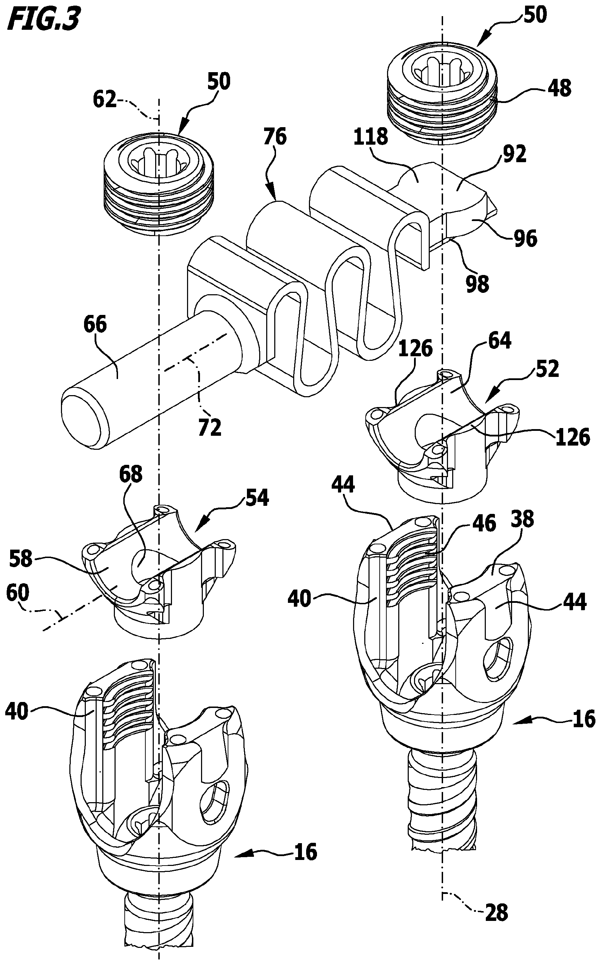

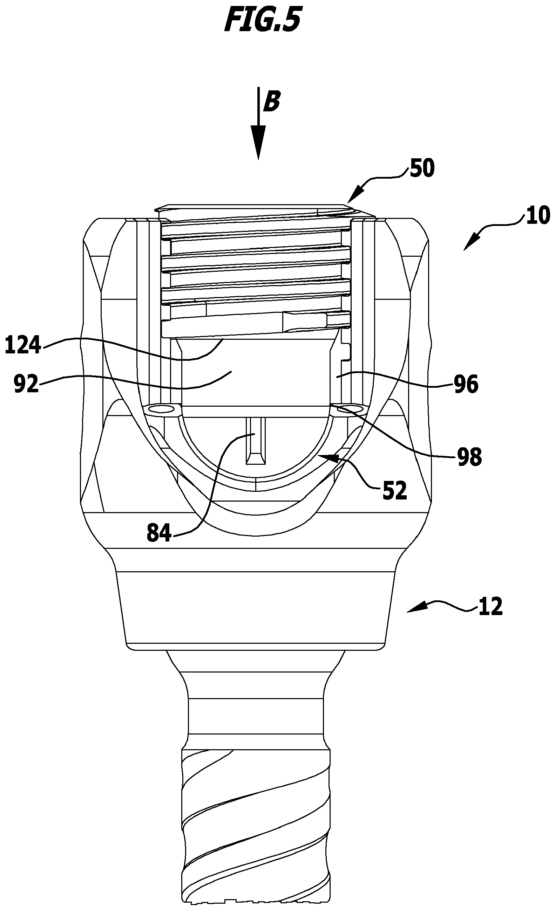

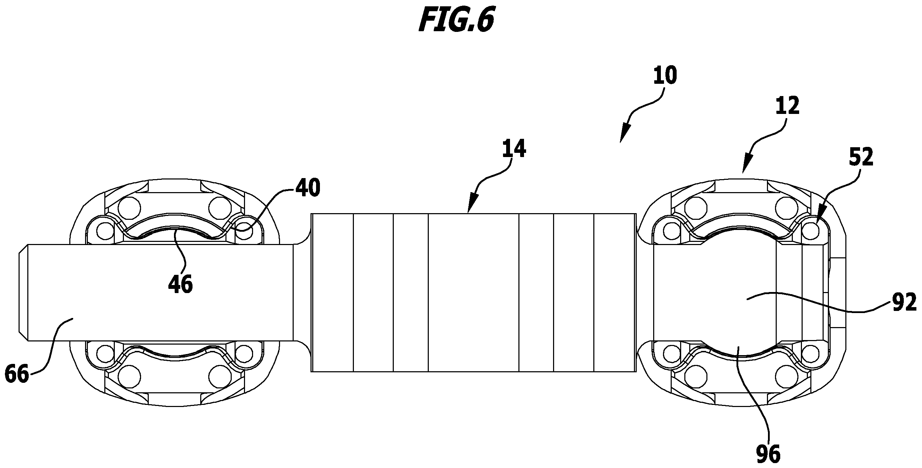

In Figur 1 ist schematisch ein Wirbelsäulenstabilisierungssystem 10 dargestellt, welches insgesamt vier Knochenbefestigungseinrichtungen 12 und zwei Verbindungselemente 14 umfasst. Die Knochenbefestigungseinrichtungen 12 sind in Form von Knochenschrauben 16 ausgebildet. In den Figuren 1 bis 6 sind beispielhaft sogenannte polyaxiale Knochenschrauben 16 dargestellt.

Die Knochenschrauben 16 sind in Pedikeln 18 von Wirbeln 20 einer Wirbelsäule 22 eingeschraubt. Zum Stabilisieren derselben werden beispielsweise in Pedikeln 18 benachbarter Wirbel 20 jeweils eine Knochenschraube 16 eingeschraubt, die dann über ein Verbindungselement 14 miteinander gekoppelt werden.

Die polyaxialen Knochenschrauben 16 umfassen einen Befestigungsteil 24 und einen Halteteil 26. Der Befestigungsteil 24 umfasst einen eine Längsachse 28 definierenden Schaft mit einem Außengewinde 32 zum Einschrauben in Knochen. Ein proximales Ende des Schafts 30 ist in Form eines kugeligen Kopfes 34 ausgebildet, auf welchem der Halteteil 26 beweglich gelagert ist. Der Halteteil 26 ist im wesentlichen hülsenförmig ausgebildet und weist distalseitig einen hohlkugeligen Sitz 36 für den Kopf 34 auf, so dass der Halteteil 26 in einer Montagestellung um einen Mittelpunkt des Kopfes 34 relativ zum Befestigungsteil 24 verschwenkbar ist. Der Halteteil 26 weist ausgehend von seinem proximalen Ende 38 zwei einander bezogen auf die Längsachse 28 diametral gegenüberliegende Einschnitte 40 auf, so dass zwei eine Verbindungselementaufnahme 42 seitlich begrenzende, bezogen auf die Längsachse 28 symmetrisch zueinander angeordnete Wandabschnitte 44 ausgebildet werden. Ausgehend vom Ende 38 ist am Halteteil 26 im Bereich der Wandabschnitte 44 ein Innengewinde 46 ausgebildet, welches zu einem Außengewinde 48 einer Fixierschraube 50 korrespondiert.

Ferner umfasst der Halteteil 26 ein Klemmglied 52 in Form eines Einsatzes 54. Dieser weist hohlkalottenförmiges distales Ende 56 auf, welches in Anlage mit dem Kopf 34 bringbar ist. Ein proximales Ende ist im Wesentlichen in Form einer halben Zylinderhülse 58 ausgebildet, deren Zylinderhülsenlängsachse 60 sich quer zu einer Haltteillängsachse 62 erstreckt. Die Zylinderhülse 58 definiert eine hohlzylindrische Anlagefläche 64 für insbesondere ein erstes, in Form eines zylindrischen Stabes ausgebildetes Ende 66 des Verbindungselements 14.

Ferner weist der Einsatz 54 eine sich koaxial zur Halteteillängsachse 62 erstreckende Durchbrechung in Form einer Bohrung 68 auf.

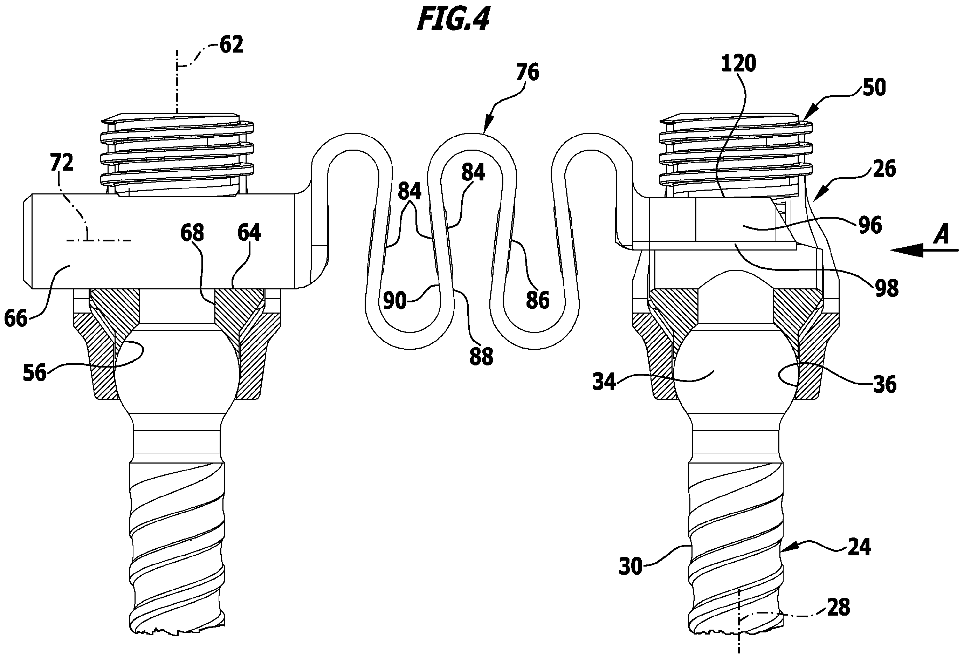

Das erste Ende 66 kann, wie schematisch in Figur 4 dargestellt, in die distalseitig von der Anlagefläche 64 begrenzte Verbindungselementaufnahme 42 eingelegt und mit der Fixierschraube 50 klemmend gesichert werden. Dabei wird gleichzeitig auch der Halteteil 26 in einer gewünschten Implantationsstellung relativ zum Befestigungsteil 24 klemmend fixiert, und zwar indem die mittels der Fixierschraube 50 eingeleitete Klemmkraft über das erste Ende 66 auf den Einsatz 54 geleitet wird, welcher wiederum auf den Kopf 34 drückt. Das Verbindungselement 14 umfasst das erste Ende 66 sowie ein zweites Ende 70 und einen zwischen diesen angeordneten oder ausgebildeten, mindestens teilweise flexiblen und eine Längsachse 72 definierenden Zwischenabschnitt 74. Dieser ist in Form eines streifenförmigen, gewundenen Blattfederelements 76 ausgebildet. Ferner weist er insgesamt fünf Ausnehmungen 78 und 80 auf, die in einer Ausnehmungsrichtung 82 quer zur Längsachse 72 seitlich geöffnet sind. Dabei sind die drei Ausnehmungen 80 in entgegengesetzter Richtung geöffnet bezogen auf die Ausnehmungen 78. Durch die Ausgestaltung als Blattfederelement 76 ist der Zwischenabschnitt 74 und damit das Verbindungselement 14 in definierter Weise elastisch.

Eine Elastizität des Zwischenabschnitts 74 kann zudem optional durch eines oder mehrere Steifigkeitsänderungselemente 84 variiert werden. Diese können beispielsweise in Form kurzer streifenförmiger Vorsprünge 86 auf einer oder beiden Oberflächen 88 beziehungsweise 90 des Blattfederelements 76 angeordnet oder ausgebildet sein. Vorzugsweise sind sie einstückig mit dem Blattfederelement 76 ausgebildet. Weitere Beispiele für die Ausbildung von Steifigkeitsänderungselementen 84 sind aus der DE 20 2010 008 865 U1 bekannt.

Das zweite Ende 70 des Verbindungselements 14 ist in Form eines Kopplungselements 92 ausgebildet zum Festlegen des Verbindungselements 14 an der Knochenbefestigungseinrichtung 12 in mindestens einer definierten Orientierung bezogen auf die Längsachse 72. Es weist eine von einem Kreiszylinder abweichende Form auf. Ferner ist es, ebenso wie das Verbindungselement 14 insgesamt, einstückig ausgebildet.

Das Kopplungselement 92 weist einen Kopplungselementgrundkörper 94 und insgesamt vier Kopplungsglieder 96 und 98 auf. Die Kopplungsglieder 96, die in Form von konvex vom Kopplungselementgrundkörper 94 weg weisenden Kopplungsvorsprüngen 102 ausgebildet sind, erstrecken sich in einer Kopplungsgliedrichtung 104, die quer zur Längsachse 72 verläuft. Die Kopplungsglieder 98 sind in Form von Rücksprüngen 100 am quaderförmigen Kopplungselementgrundkörper 94 ausgebildet und definieren eine Kopplungsgliedrichtung 106, die parallel zur Längsachse 72 verläuft.

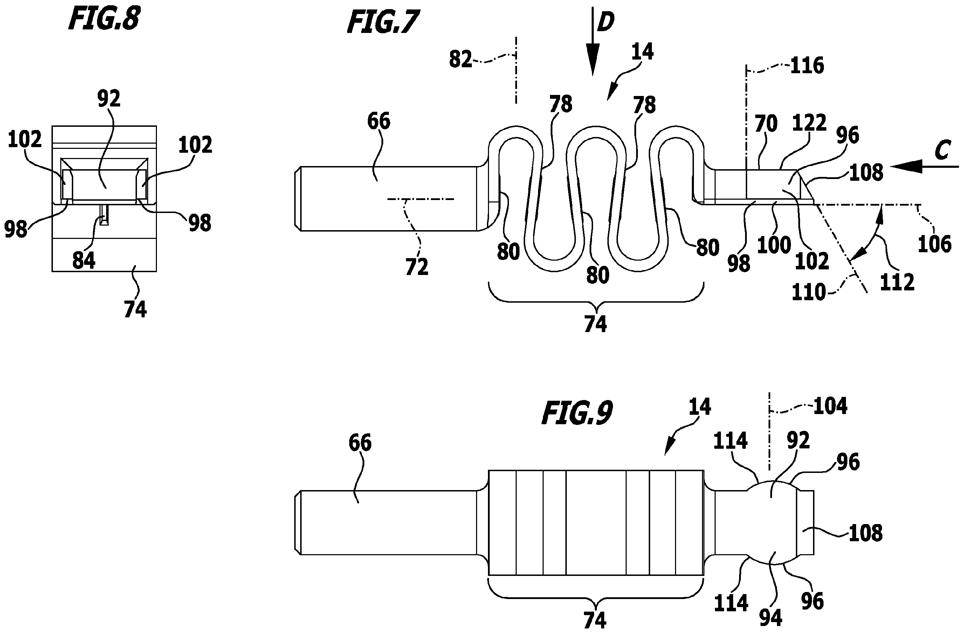

Ferner ist eine vom Zwischenabschnitt 74 weg weisende Endfläche 108 des Kopplungselements 92 bezogen auf die Längsachse 72 geneigt. Die Endfläche 108 definiert eine Endflächenebene 110, die mit der Längsachse 72 einen Endflächenneigungswinkel 112 einschließt, welcher vorzugsweise in einem Bereich von etwa 30° bis etwa 90° liegt. Bei dem in den Figuren 1 bis 9 schematisch dargestellten Verbindungselement 14 beträgt der Endflächenneigungswinkel 112 etwa 60°.

Die vom Kopplungselementgrundkörper 94 weg weisenden, von den Kopplungsgliedern 96 definierten Seitenflächen 114 bilden gemeinsam einen Ausschnitt eines Kreiszylinders, dessen Längsachse eine Vorzugsrichtung 116 des Kopplungselements 92 definiert. Diese verläuft quer zur Längsachse 72. Ein durch die Kopplungsglieder 96 definierter Durchmesser ist an einen Innendurchmesser des Innengewindes 46 angepasst, so dass das Kopplungselement 92 in definierter Orientierung bezogen auf die Längsachse 72 an der Knochenschraube 16 festlegbar ist.

Ein Schwerpunkt des Kopplungselements 92 ist von der Längsachse 72 beabstandet.

Eine Oberseite 118 des Kopplungselementgrundkörpers 94 definiert einen ebenen Flächenabschnitt 120, welcher eine parallel zur Längsachse 72 verlaufende Ebene definiert. Der Flächenabschnitt 120 bildet einen Klemmflächenabschnitt 122 für ein distales Ende 124 der Fixierschraube 50. So kann zum Klemmen des Kopplungselements 92 in der Verbindungselementaufnahme 42 das Ende 124 im Wesentlichen flächig an den Klemmflächenabschnitt 122 gedrückt werden.

Die Rücksprünge 100 sind derart angeordnet und bemessen, dass sie auf parallel zur Längsachse 72 verlaufenden, in proximaler Richtung weisenden Kanten 126 des Einsatzes 54 aufliegen.

Das Verbindungselement 14 definiert eine Vorzugsrichtung 116, kann also genau auf eine definierte Weise in der Verbindungselementaufnahme 42 festgelegt werden. Durch die Kopplungsglieder 96 wird das Verbindungselement 14 zudem parallel zur Längsachse 72 relativ zur Knochenschraube 16 gesichert, kann also selbst dann, wenn sich die Fixierschraube 50 etwas lösen sollte, parallel zur Längsachse 72 relativ zur Knochenschraube 16 nicht bewegt werden. Eine Rotation des Verbindungselements 14 um die Längsachse 72 relativ zur Knochenschraube 16 ist ebenfalls nicht möglich.

Nachfolgend werden weitere Ausführungsbeispiele von Verbindungselementen näher beschrieben, die sich im Wesentlichen in der Ausgestaltung ihrer Kopplungselemente unterscheiden. Daher werden identische oder vergleichbare Teile und Elemente mit denselben Bezugszeichen bezeichnet.

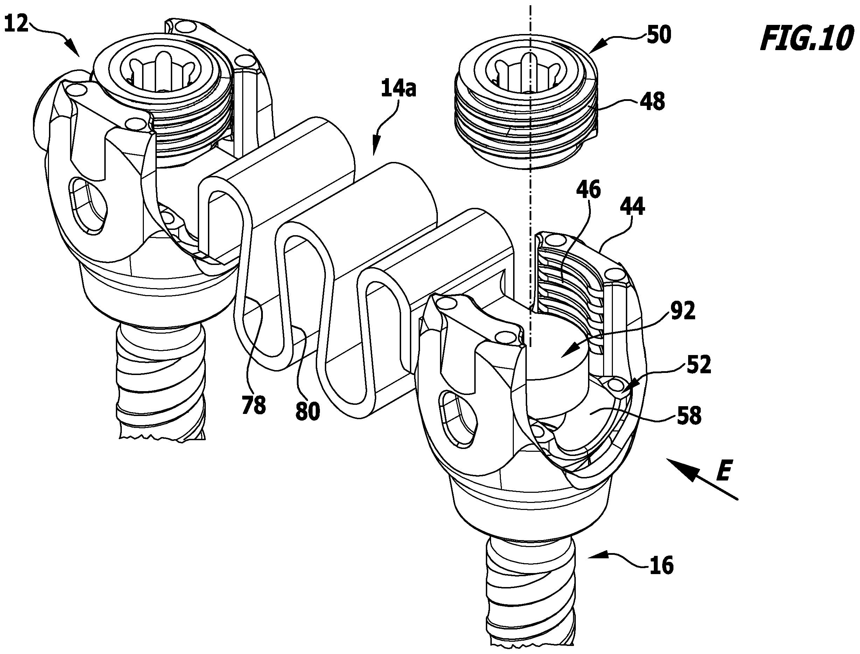

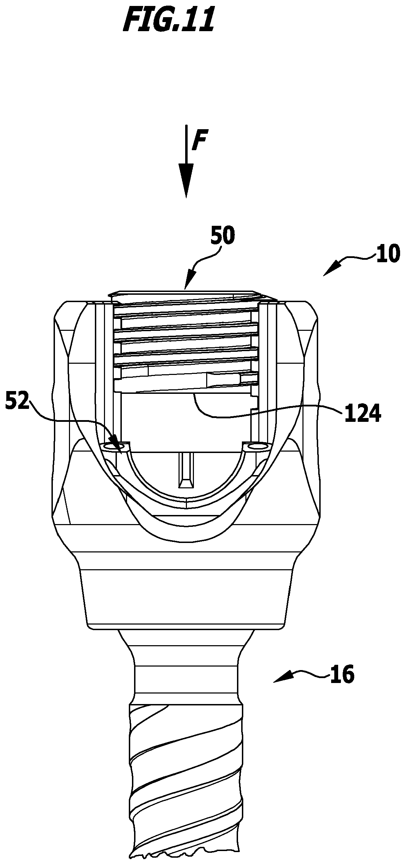

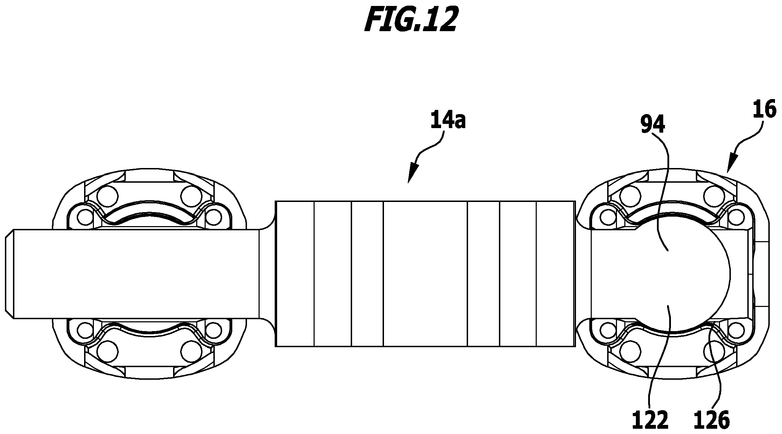

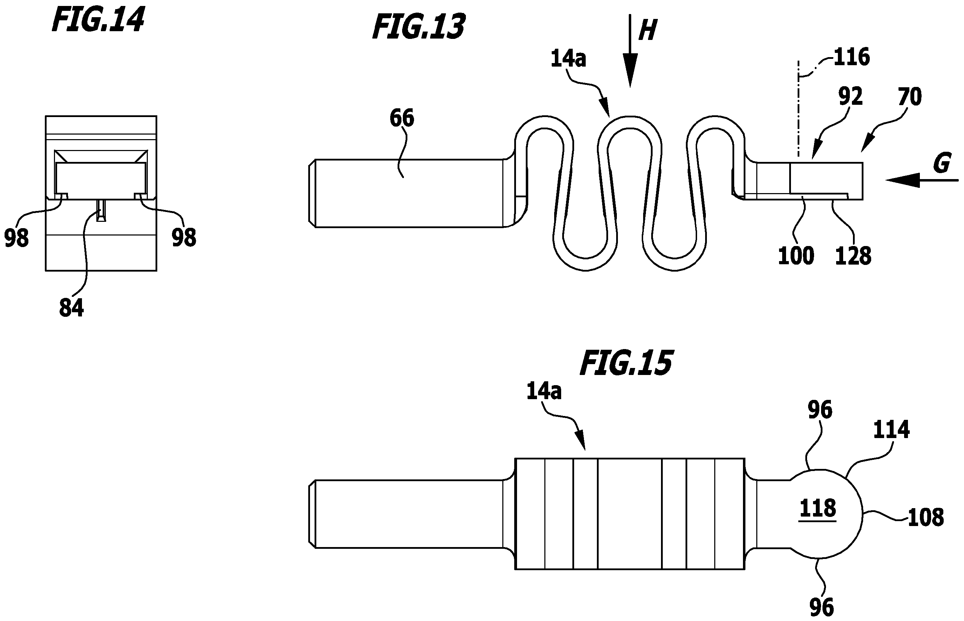

In den Figuren 10 bis 15 ist schematisch ein zweites Ausführungsbeispiel eines Verbindungselements dargestellt und insgesamt mit den Bezugszeichen 14a bezeichnet. Es unterscheidet sich im Aufbau vom Verbindungselement 14 durch die Form des Kopplungselements 92.

Dieses weist ebenfalls einen quaderförmigen Kopplungselementgrundkörper 94 auf, von dem sich quer zur Längsachse 72 zwei Kopplungsglieder 96 seitlich weg erstrecken. Eine vom Zwischenabschnitt 74 weg weisende Endfläche 108 ist abgerundet und bildet mit den Kopplungsgliedern 96 zusammen einen Teil einer zylindrischen Scheibe aus, die eine zylindrische Seitenfläche 114 aufweist. Ein durch die Seitenfläche 114 definierter Durchmesser des Kopplungselements 92 entspricht einem Innendurchmesser des Innengewindes 46, so dass das Kopplungselement 92 zur axialen Sicherung des Verbindungselements 14a in Richtung der Längsachse 72 relativ zur Knochenschraube 16 in die Verbindungselementaufnahme 42 einlegbar ist.

Ferner sind am Kopplungselementgrundkörper 94 zwei weitere parallel zur Längsachse 72 verlaufende Kopplungsglieder 98 in Form von Rücksprüngen 100 ausgebildet. Sie liegen, wenn das zweite Ende 70 in die Verbindungselementaufnahme 42 eingelegt ist, auf den Kanten 126 des Einsatzes 54 auf.

Das Kopplungselement 92 definiert eine Vorzugsrichtung 116, die quer zur Längsachse 72 verläuft. Eine Oberseite 118 des Kopplungselements 92 bildet einen ebenen Klemmflächenabschnitt 122 für das distale Ende 124 der Klemmschraube 50.

Das Verbindungselement 14a kann aufgrund seiner Ausgestaltung sowohl parallel zur Längsachse 72 als auch in Umfangsrichtung desselben definiert zur Knochenschraube 16 orientiert werden.

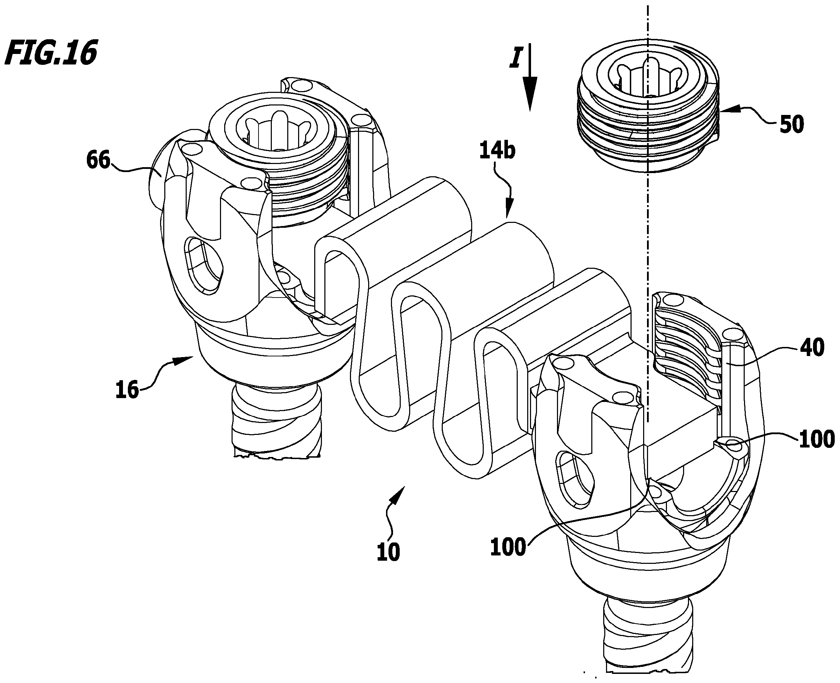

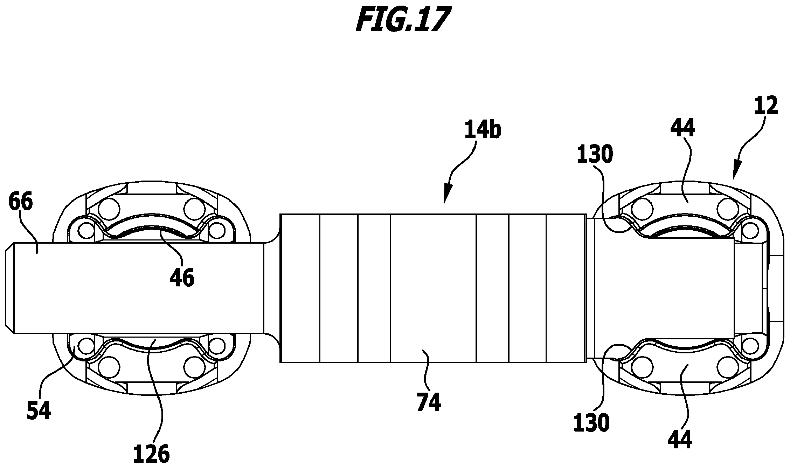

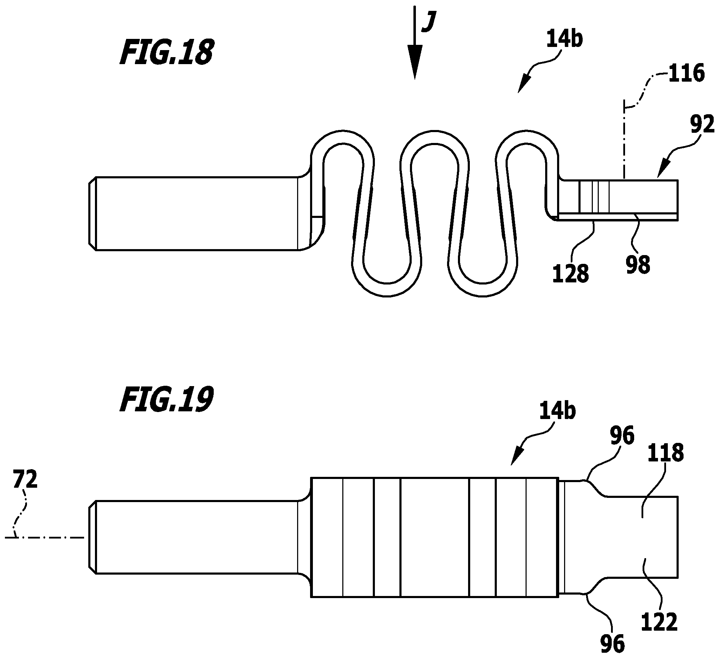

In den Figuren 16 bis 19 ist schematisch ein drittes Ausführungsbeispiel eines Verbindungselements dargestellt und insgesamt mit dem Bezugszeichen 14b bezeichnet. Es unterscheidet sich in der Ausgestaltung des Kopplungselements 92 vom Verbindungselement 14.

Das Kopplungselement 92 weist einen quaderförmigen Kopplungselementgrundkörper 94 auf, dessen Oberseite 118 einen ebenen Klemmflächenabschnitt 122 definiert. Eine vom Zwischenabschnitt 74 weg weisende Endfläche 118 ist ebenfalls eben und verläuft quer zur Längsachse 72.

Am Kopplungselementgrundkörper 94 sind zwei seitlich, also quer zur Längsachse 72 vorspringende Kopplungsglieder 96 ausgebildet sowie zwei parallel zur Längsachse 72 verlaufende Kopplungsglieder 98. Die Kopplungsglieder 98 sind in der Unterseite 128 des Kopplungselementgrundkörpers 94 in Form von Rücksprüngen 100 ausgebildet, die sich über die ganze Länge des Kopplungselements 92 parallel zur Längsachse 72 erstrecken. Sie sind so dimensioniert und bemessen, dass sie wiederum auf den Kanten 126 des Einsatzes 54 aufliegen.

Der Kopplungselementgrundkörper 94 weist eine Breite auf, die einer lichten Weite zwischen den beiden Wandabschnitten 44 entspricht, so dass das Kopplungselement 72 in die Verbindungselementaufnahme 42 einlegbar ist.

Die Kopplungsglieder 96 liegen an den Wandabschnitten an von diesen weg weisend konkav gekrümmten Seitenflächen 130 an, die bei eingelegtem Kopplungselement 92 im Wesentlichen in Richtung auf den Zwischenabschnitt 74 hin weisen. Die Kopplungsglieder 96 sind in ihrer Form an die Seitenflächen 130 angepasst, so dass eine flächige Anlage des Kopplungselements 92 an die Seitenflächen 130 möglich ist. Die Seitenflächen 130 bilden so einen Anschlag für das Verbindungselement 14b, so dass es nur so weit parallel zur Längsachse 72 in Richtung auf die Knochenschraube 16 hin bewegt werden kann, bis die Kopplungsglieder 96 an den Seitenflächen 130 anschlagen. Eine Rotation des Kopplungselements 92 in der Verbindungselementaufnahme 42 ist nicht möglich. Insgesamt definiert das Kopplungselement 92 eine Vorzugsrichtung 116, die quer zur Längsachse 72 verläuft.

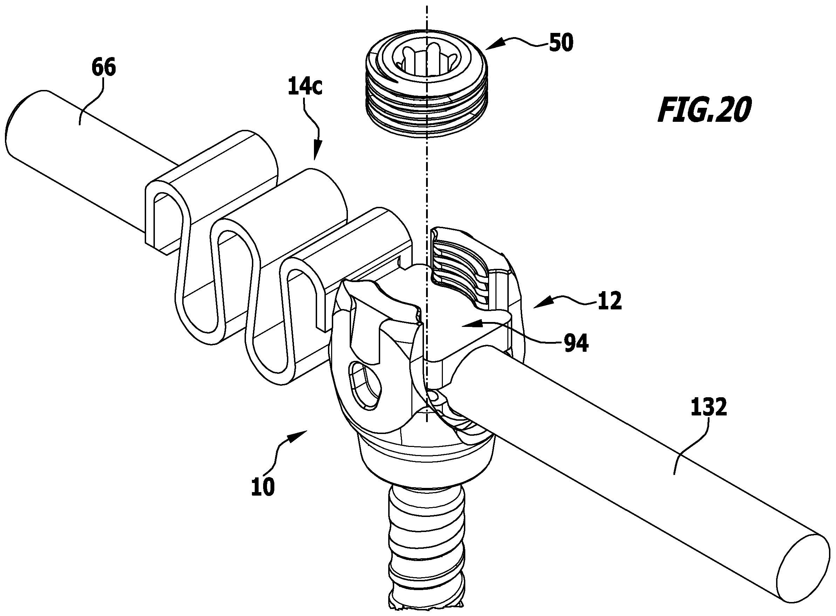

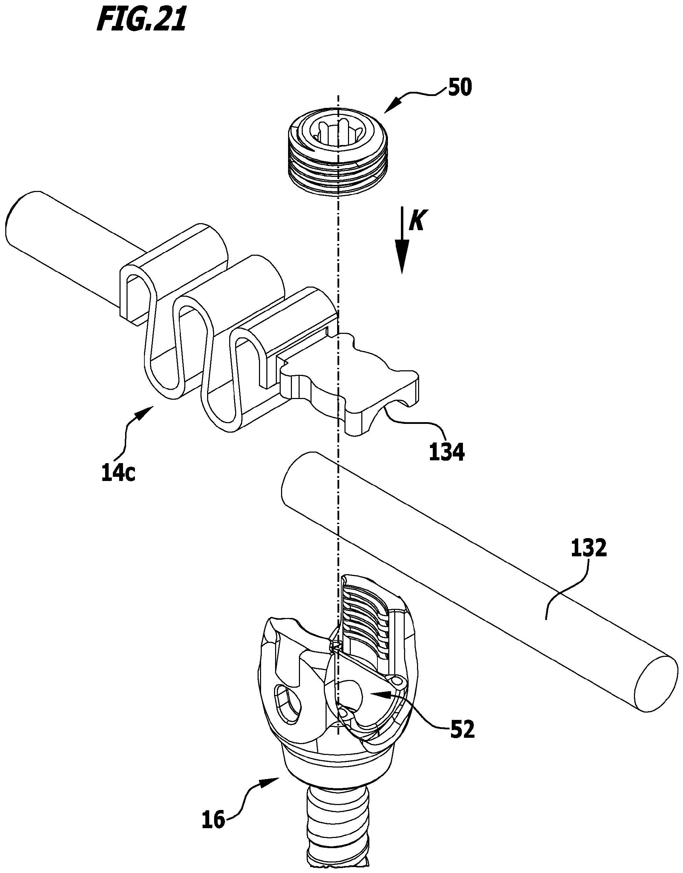

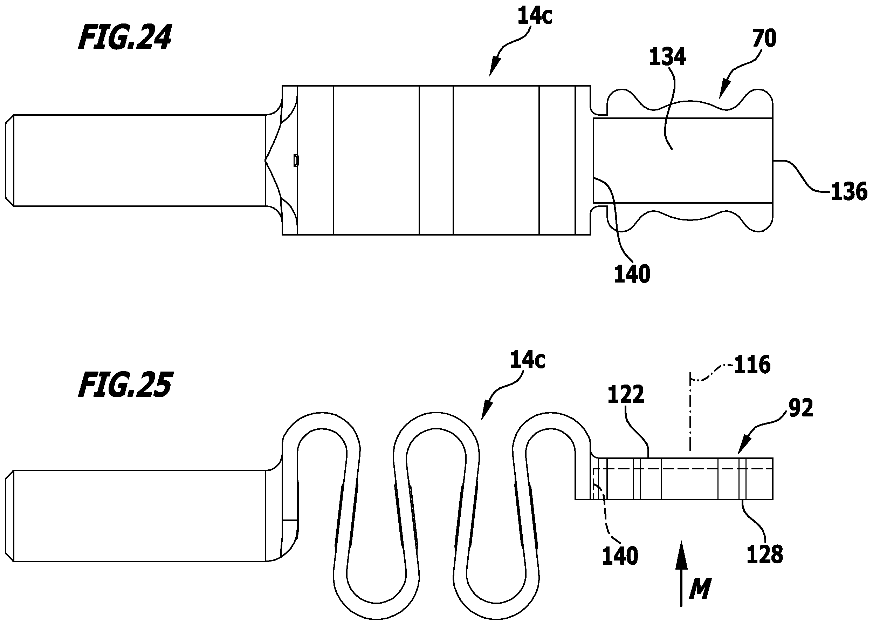

In den Figuren 20 bis 25 ist schematisch ein viertes Ausführungsbeispiel eines insgesamt mit dem Bezugszeichen 14c bezeichneten Verbindungselements dargestellt. Es unterscheidet sich in seinem Aufbau vom Verbindungselement 14 durch das ein zweites Ende bildendes Kopplungselement 92. Das Kopplungselement 92 weist einen im Wesentlichen quaderförmigen Kopplungselementgrundkörper 94 auf, welcher zwei in Form von Kopplungsvorsprüngen 102 ausgebildete Kopplungsglieder 96 aufweist, die quer zur Längsachse 72 seitlich am Kopplungselementgrundkörper 94 abstehen. Die Kopplungsglieder 96 definieren einen Durchmesser, welcher einem Innendurchmesser des Innengewindes 46 entspricht.

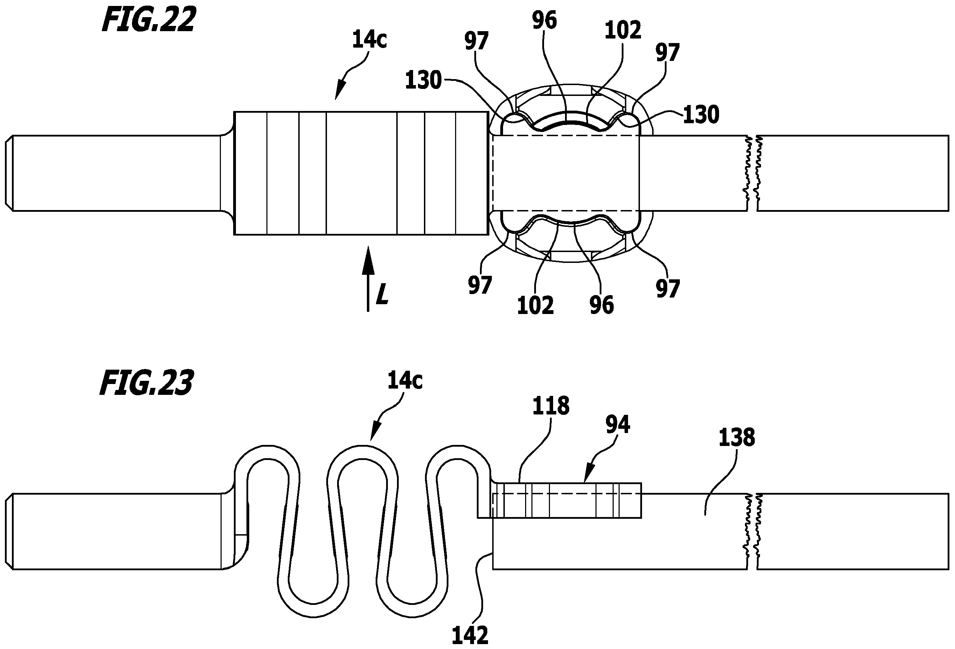

An die Kopplungsglieder 96 schließen sich sowohl auf einer dem Zwischenabschnitt 74 zugewandten als auch auf einer diesem abgewandten Seite an jedes Kopplungsglied 96 zwei Kopplungsglieder 97 an, die ebenfalls in Form von Kopplungsvorsprüngen ausgebildet sind. Sie stehen etwas weiter vom Kopplungselementgrundkörper 94 ab als die Kopplungsglieder 96. Ferner weisen sie eine äußere Kontur auf, die an die konkaven Seitenflächen 130 der Wandabschnitte 44 angepasst ist. So kann das Kopplungselement 92, wie beispielsweise in Figur 22 gut zu erkennen, im Wesentlichen formschlüssig von oben in die Verbindungselementaufnahme 42 eingelegt werden.

Eine Oberseite 118 des Kopplungselements 92 ist eben und bildet einen Klemmflächenabschnitt 122 für das distale Ende 124 der Fixierschraube 50.

Das Kopplungselement 92 weist ferner eine Verbindungselementanlagefläche 134 auf zum Anlegen an ein weiteres Verbindungselement, beispielsweise in Form eines Verbindungsstabs 132, welcher langgestreckt und in Form eines Kreiszylinders ausgebildet ist. Die Verbindungselementanlagefläche 134 ist in Form einer hohlzylindrischen Wandfläche 136 ausgebildet, an welche eine Außenfläche 138 des Verbindungsstabs 132 flächig angelegt werden kann. Vom Zwischenabschnitt 74 in Richtung auf das Kopplungselement 92 weisend ist eine Anschlagfläche 140 ausgebildet, an welcher eine Endfläche 142 des Verbindungsstabs 132 anschlagen kann.

Die besondere Ausgestaltung des Kopplungselements 92 ermöglicht es, dieses zusammen mit dem Verbindungsstab 132 an der Knochenschraube 16 festzulegen. Der Verbindungsstab 132 wird dann klemmend zwischen dem Kopplungselement 92 und dem Einsatz 54 gehalten, das Kopplungselement 92 zwischen dem Verbindungsstab 132 und der Fixierschraube 50.

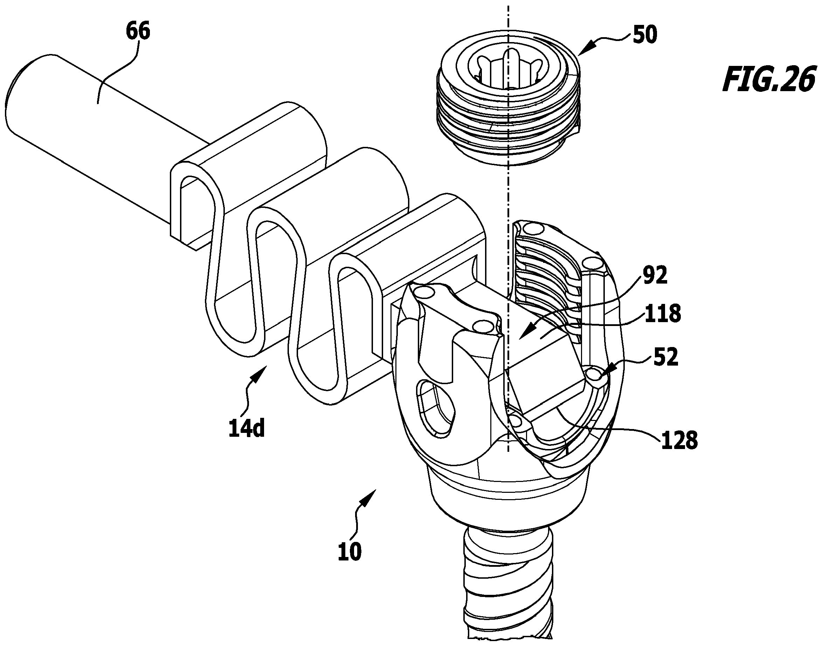

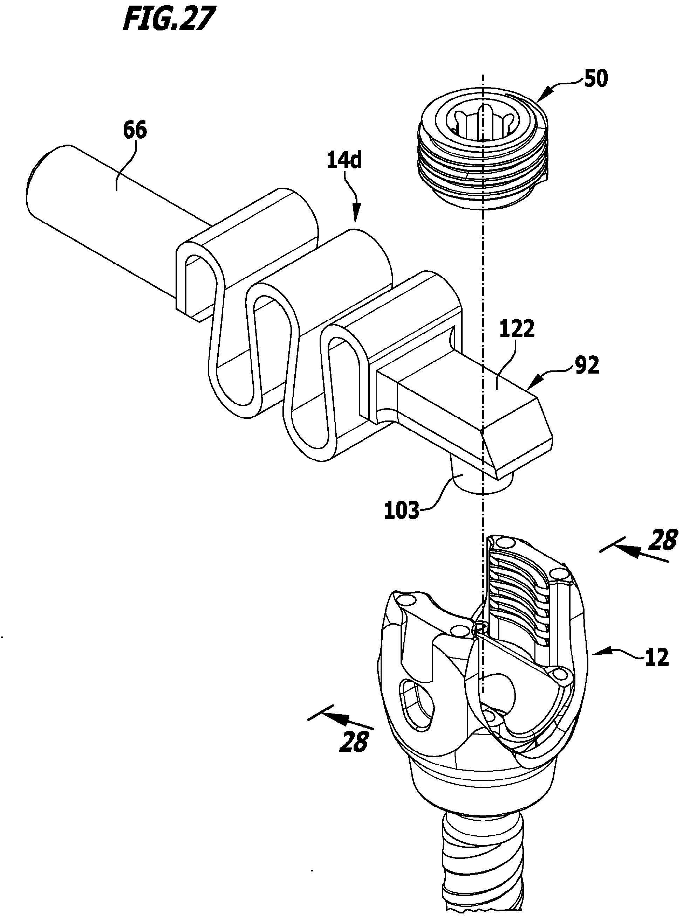

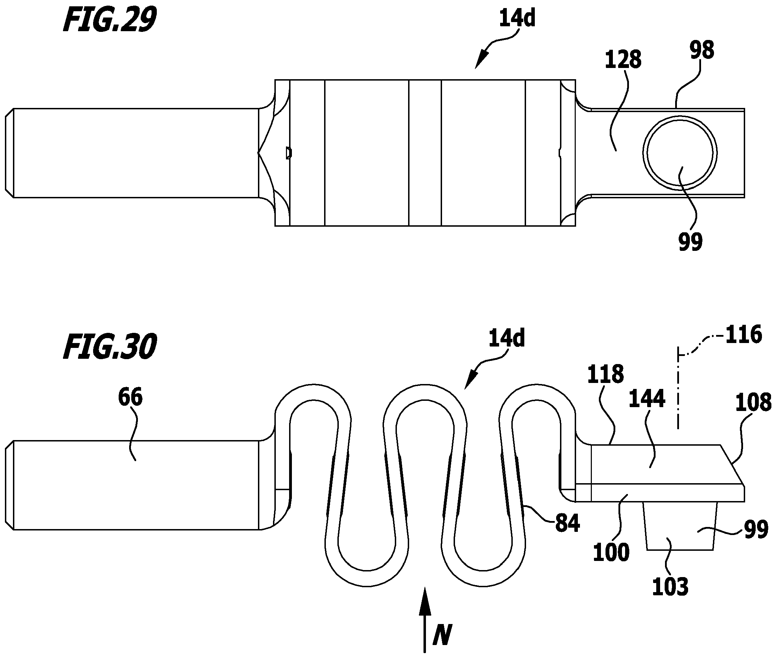

In den Figuren 26 bis 30 ist schematisch ein insgesamt mit dem Bezugszeichen 14d bezeichnetes fünftes Ausführungsbeispiel eines Verbindungselements dargestellt. Es unterscheidet sich in seinem Aufbau vom Verbindungselement 14 durch die Ausgestaltung des Kopplungselements 92.

Das Kopplungselement 92 weist wiederum einen quaderförmigen Grundkörper auf mit einer vom Zwischenabschnitt 74 weg weisenden, relativ zur Längsachse 72 geneigten Endfläche 108. Eine Oberseite 118 des Kopplungselements 92 ist eben und bildet einen Klemmflächenabschnitt 122 für das distale Ende 124 der Fixierschraube 50.

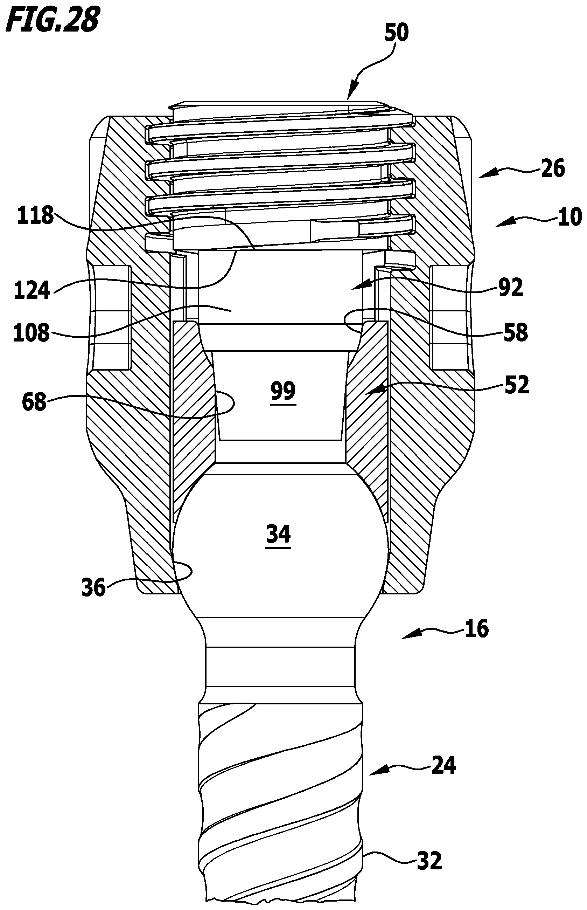

Im Wesentlichen parallel zur Längsachse 72 verlaufende Grundkörperseitenflächen 144 sind im Übergang zur Unterseite 128 gekrümmt und bilden Kopplungsglieder 98 in Form von Rücksprüngen 100. Eine Krümmung der Kopplungsglieder 98 entspricht einem von der Anlagefläche 64 des Einsatzes 54 definierten Innendurchmesser, so dass die Kopplungsglieder 98 im Wesentlichen flächig an der Anlagefläche 64 anliegen. Dies ist beispielhaft in Figur 28 gut zu erkennen.

Von der Unterseite 128 steht ein weiteres Kopplungsglied 99 quer zur Längsachse 72 ab und definiert eine Vorzugsrichtung 116. Das Kopplungsglied 99 ist in Form eines Kopplungsvorsprungs 103 ausgebildet und weist die Form eines sich von der Unterseite 128 weg erstreckenden Konus auf. Der Konus ist so bemessen, dass er in die Bohrung 68 des Einsatzes 54 einführbar ist.

Durch die besondere Ausgestaltung des Kopplungselements 92 ist dieses sowohl parallel zur Längsachse 72 als auch gegen eine Rotation um dieselbe an der Knochenschraube 16 sicherbar.

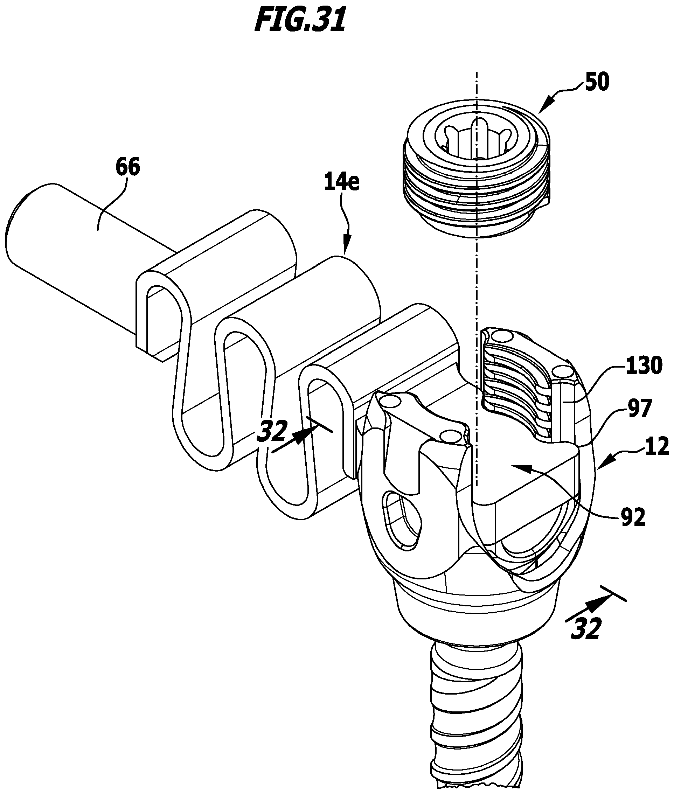

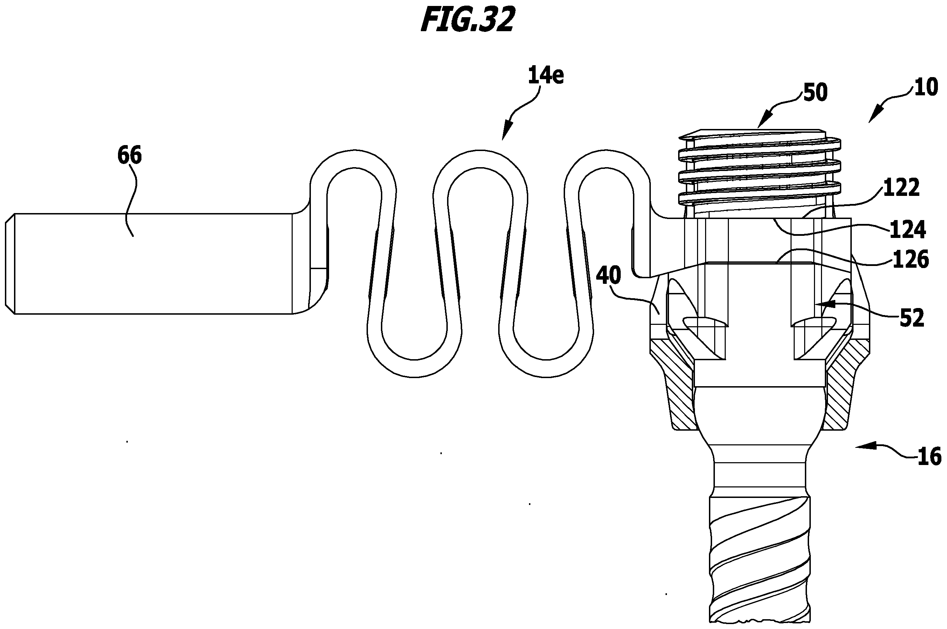

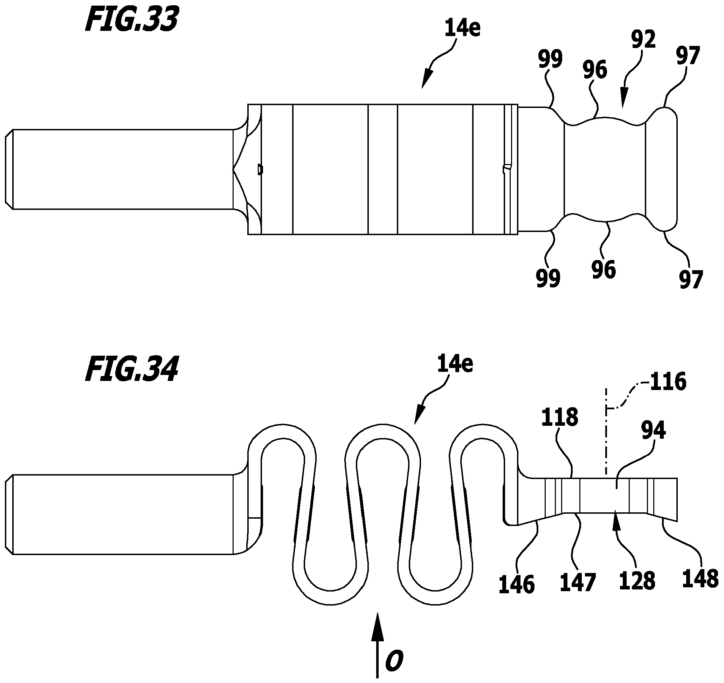

In den Figuren 31 bis 34 ist schematisch ein sechstes Ausführungsbeispiel eines insgesamt mit den Bezugszeichen 14e bezeichneten Verbindungselements dargestellt. Es unterscheidet sich in seinem Aufbau vom Verbindungselement 14 durch die Ausgestaltung des Kopplungselements 92.

Das Kopplungselement 92 weist einen im Wesentlichen quaderförmigen Kopplungselementgrundkörper 94 auf mit zwei seitlich abstehenden Kopplungsgliedern 96. Diese sind vom Kopplungselementgrundkörper 94 weg weisend konvex gekrümmt und definieren einen Durchmesser, welcher einem Innendurchmesser des Innengewindes 46 entspricht.

Vom Zwischenabschnitt 74 weg weisend schließt sich an die Kopplungsglieder 96 jeweils ein Kopplungsglied 97 in Form eines weiteren Kopplungsvorsprungs an. Die Kopplungsglieder 97 weisen eine Außenkontur auf, so dass sie mindestens abschnittsweise im Wesentlichen flächig an die Seitenflächen 130 der Wandabschnitte 44 anlegbar sind.

Proximalseitig schließen sich an die Kopplungsglieder 96 weitere Kopplungsvorsprünge 99 an, die im Wesentlichen in Form der Kopplungsvorsprünge 96 des Verbindungselements 14b ausgebildet sind. Die Kopplungsglieder 96 weisen eine Außenkontur auf, die zumindest teilweise ein flächiges Anliegen an die Seitenflächen 130 der Wandabschnitte 44 ermöglicht.

Eine Oberseite 118 des Kopplungselements 92 bildet einen ebenen Klemmflächenabschnitt 122 für das distale Ende 124 der Fixierschraube 50. Die Unterseite 128 des Kopplungselements 92 wird gebildet durch drei relativ zueinander geneigte Kopplungselementgrundkörperseitenflächen 146, 147 und 148. Die Kopplungselementgrundkörperseitenfläche 147 verläuft parallel zur Oberseite 148 und erstreckt sich im Wesentlichen im Bereich der Kopplungsglieder 96. Die Kopplungselementgrundkörperseitenfläche 148 ist relativ zur Kopplungselementgrundkörperseitenfläche 147 geneigt und schließt mit dieser einen stumpfen Winkel ein. Die Kopplungselementgrundkörperseitenfläche 148 erstreckt sich im Wesentlichen im Bereich der Kopplungsglieder 97.

Die Kopplungselementgrundkörperseitenfläche 146 erstreckt sich im Wesentlichen im Bereich der Kopplungsglieder 99 und schließt mit der Kopplungselementgrundkörperseitenfläche 147 einen stumpfen Winkel ein. Insgesamt bilden die drei Kopplungselementgrundkörperseitenflächen 146, 147 und 148 zusammen eine im Wesentlichen konkav gekrümmte Unterseite 128 des Kopplungselements 92.

Das Kopplungselement 92 definiert eine Vorzugsrichtung 116, die quer zur Längsachse 72 verläuft.

Die Ausgestaltung des Kopplungselements 92 ermöglicht es, das Verbindungselement 14e sowohl parallel zur Längsachse 72 als auch gegen eine Rotation um diese an der Knochenschraube 16 mit der Fixierschraube 50 zu sichern.

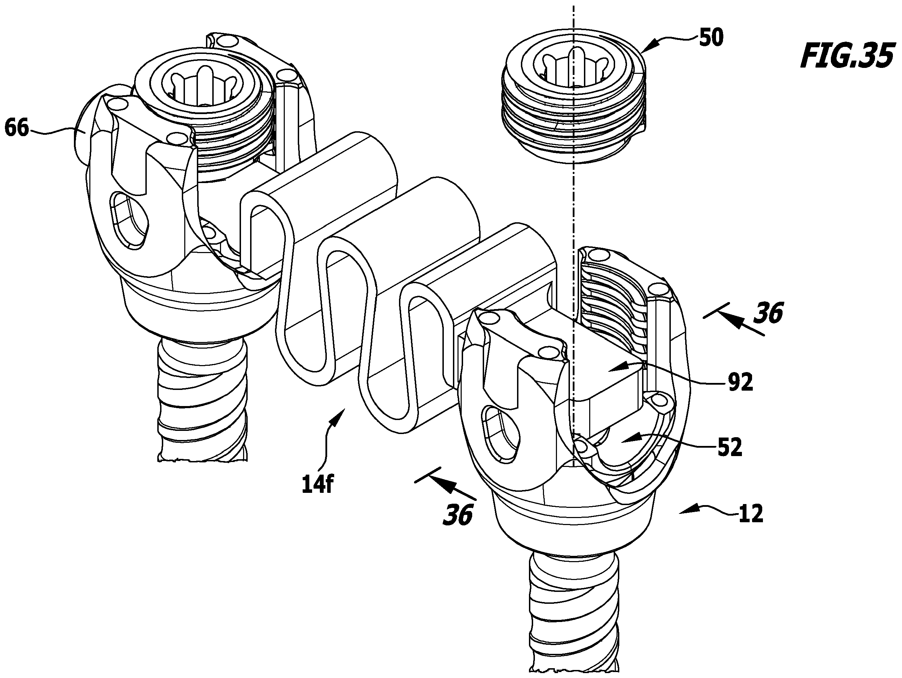

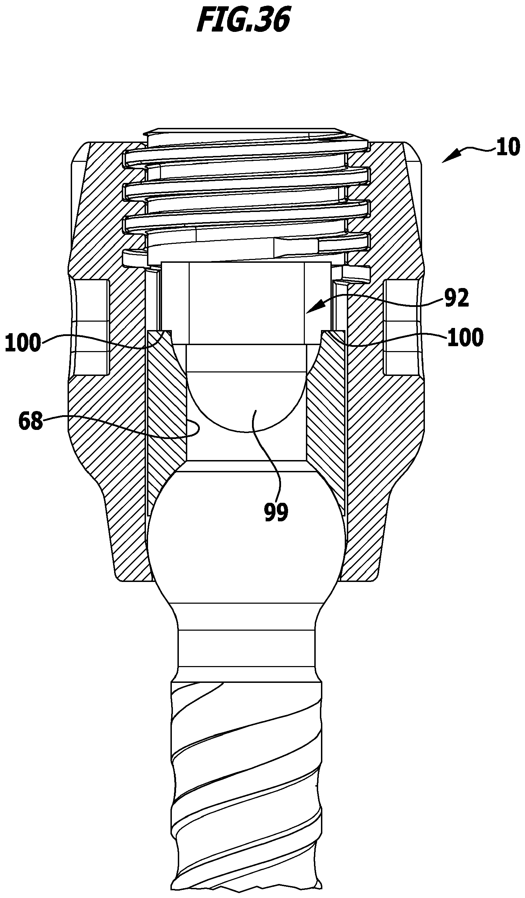

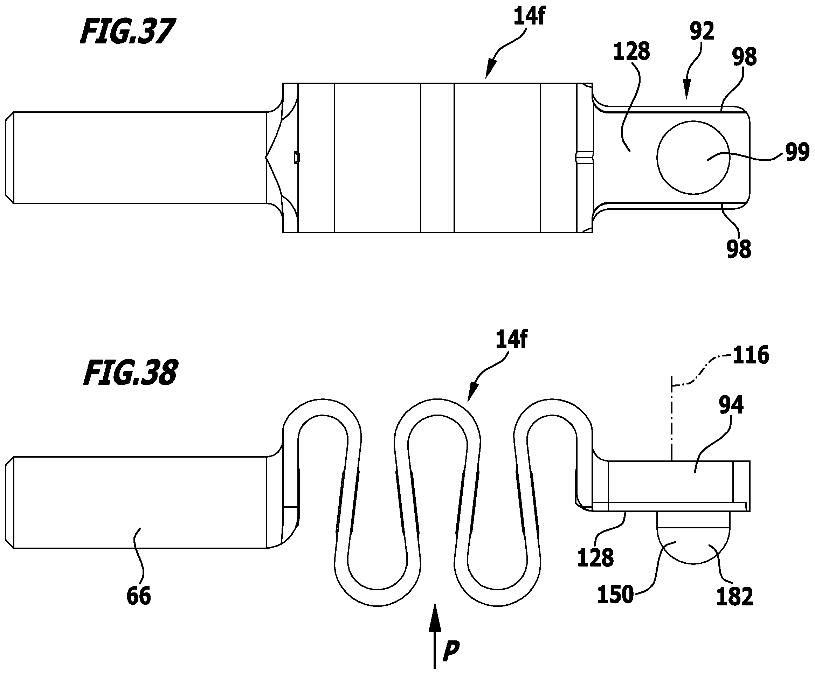

In den Figuren 35 bis 38 ist schematisch ein insgesamt mit dem Bezugszeichen 14f bezeichnetes siebtes Ausführungsbeispiel eines Verbindungselements dargestellt. Es unterscheidet sich vom Verbindungselement 14 durch die Ausgestaltung des Kopplungselements 92.

Der Kopplungselementgrundkörper 94 ist im Wesentlichen quaderförmig ausgebildet und weist an seiner Unterseite 128 zwei parallel zur Längsachse verlaufende Kopplungsglieder 98 auf, die in Form von Rücksprüngen 100 ausgebildet sind, so dass das Kopplungselement 92 mit den Rücksprüngen 100 an die Kanten 126 des Einsatzes 54 anlegbar ist. Eine Breite des Grundkörpers entspricht im Wesentlichen einer lichten Weite der Wandabschnitte 44.

Von der Unterseite 128 steht quer zur Längsachse 72 ein Kopplungsglied 99 ab, welches im Wesentlichen zylindrisch ausgebildet ist und einen Außendurchmesser definiert, welcher einem Innendurchmesser der Bohrung 68 des Einsatzes 54 entspricht. Ein vom Kopplungselementgrundkörper 94 weg weisendes Ende 150 des Kopplungsglieds 99 ist in Form einer Halbkugel 152 ausgebildet.

Das Kopplungselement 92 definiert somit eine Vorzugsrichtung 116 und ermöglicht es, das Verbindungselement 14f in einer Richtung parallel zur Längsachse 72 sowie gegen eine Rotation um die Längsachse 72 mittels der Fixierschraube 50 an der Knochenschraube 16 zu sichern.

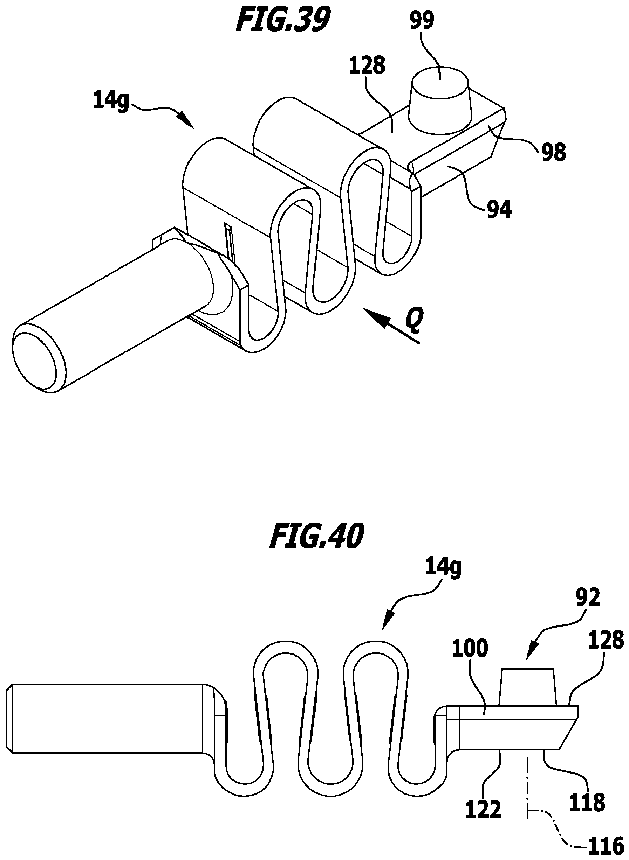

In den Figuren 39 und 40 ist ein insgesamt mit den Bezugszeichen 14g bezeichnetes achtes Ausführungsbeispiel eines Verbindungselements schematisch dargestellt. Es unterscheidet sich vom Verbindungselement 14 durch die Ausgestaltung des Kopplungselements 92.

Das Kopplungselement 92 weist einem im Wesentlichen quaderförmigen Grundkörper 94 auf, mit zwei parallel zur Längsachse 72 verlaufenden Kopplungsgliedern 98, die in Form von Rücksprüngen 100 ausgebildet sind. Eine Oberseite 118 des Kopplungselements 92 bildet einen ebenen Klemmflächenabschnitt 122 für das distale Ende 124 der Fixierschraube 50.

Von der Unterseite 128 des Kopplungselementgrundkörpers 94 steht ein Kopplungsglied 99 ab, welches in Form eines Kegels mit abgeschnittener Spitze ausgebildet ist. Das Kopplungsglied 99 ist so bemessen, dass es insbesondere in die Bohrung 68 des Einsatzes 54 einführbar ist.

Das Kopplungselement 92 definiert eine Vorzugsrichtung 116 und ermöglicht es, das Verbindungselement 14g sowohl parallel zur Längsachse 72 als auch gegen eine Rotation um die Längsachse 72 an einer Knochenschraube 16 mit der Fixierschraube 50 festzulegen.

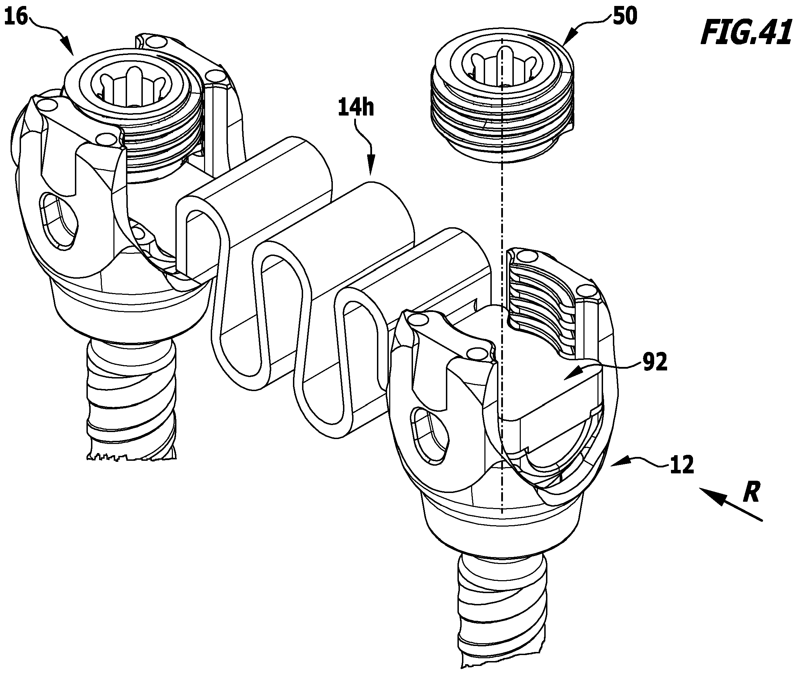

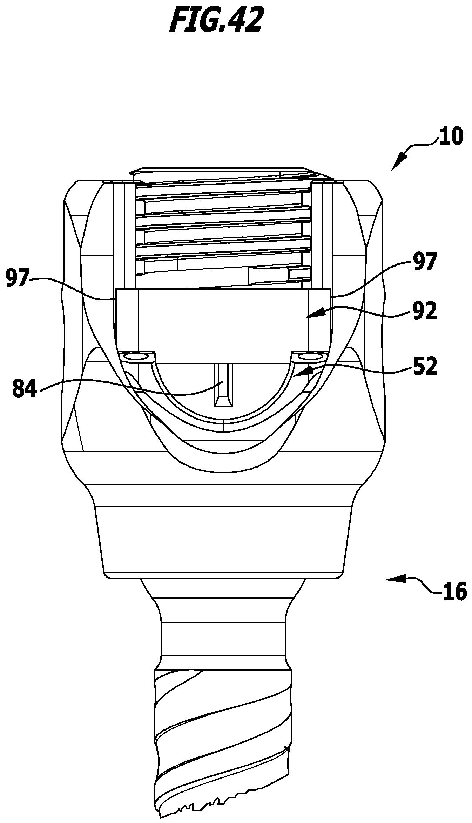

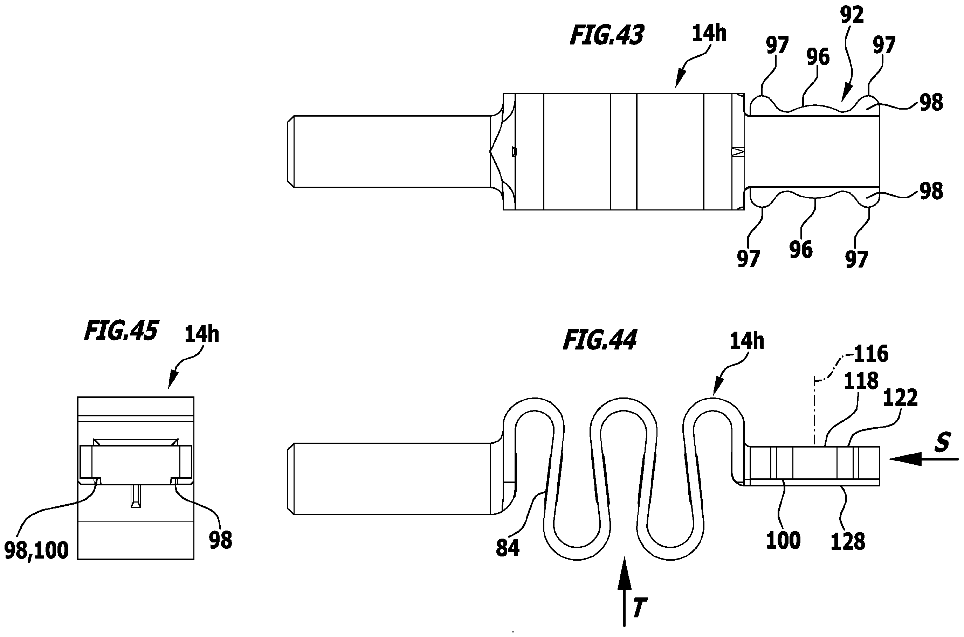

In den Figuren 41 bis 45 ist schematisch ein neuntes Ausführungsbeispiel eines insgesamt mit den Bezugszeichen 14h bezeichneten Verbindungselements schematisch dargestellt. Dieses unterscheidet sich in seinem Aufbau vom Verbindungselement 14 durch die Ausgestaltung des Kopplungselements 92.

Das Kopplungselement 92 entspricht in seinem Aufbau im Wesentlichen dem Kopplungselement 92 des Verbindungselements 14c. Es umfasst in analoger Weise Kopplungsglieder 96 und 97 und weist eine Oberseite 118 auf, die einen ebenen Klemmflächenabschnitt 122 bildet. Eine Unterseite 128 des Kopplungselements 92 ist ebenfalls eben. Anders als mit Kopplungselement 92 des Verbindungselements 14c sind beim Verbindungselement 14h jedoch parallel zur Längsachse 72 verlaufende Kopplungsglieder 98 in Form von Rücksprüngen 100 ausgebildet, die so angeordnet und bemessen sind, dass sie auf den Kanten 126 des Einsatzes 54 aufliegen, wenn das Kopplungselement 92 in die Verbindungselementaufnahme 42 eingreift.

Das Kopplungselement 92 definiert eine Vorzugsrichtung 116 und kann mit der Fixierschraube 50 in der Verbindungselementaufnahme 42 sowohl gegen eine Bewegung parallel zur Längsachse 72 als auch gegen eine Rotation um die Längsachse 72 gesichert werden.

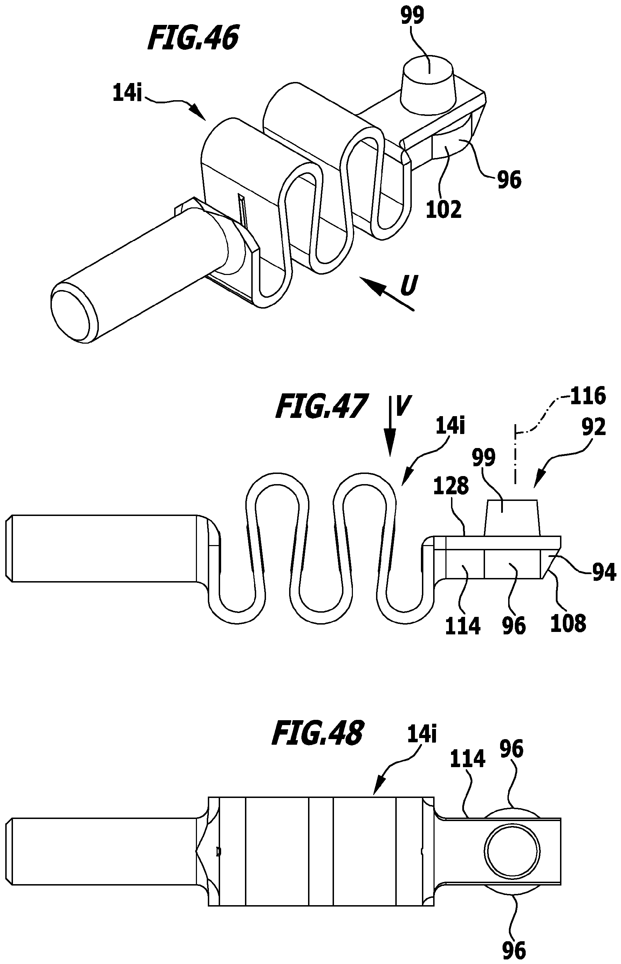

In den Figuren 46 bis 48 ist schematisch ein insgesamt mit dem Bezugszeichen 14i bezeichnetes zehntes Ausführungsbeispiel eines Verbindungselements schematisch dargestellt. Es unterscheidet sich vom Verbindungselement 14 durch die Ausgestaltung des Kopplungselements 92. Das Kopplungselement 92 ist im Wesentlichen identisch mit dem Kopplungselement 92 des Verbindungselements 14d ausgebildet. Es unterscheidet sich von diesem dadurch, dass voneinander weg weisende Seitenflächen 114 des Kopplungselementgrundkörpers zwei in diametraler Richtung voneinander weg weisend vorstehende Kopplungsglieder 96 in Form von Kopplungsvorsprüngen 102 tragen. Die Kopplungsglieder 96 definieren einen Außendurchmesser, welcher einem Innendurchmesser des Innengewindes 46 entspricht. Das Kopplungselement 92 des Verbindungselements 14i kann somit ähnlich wie das Verbindungselement 14d in die Verbindungselementaufnahme 42 eingesetzt werden, wobei zusätzlich noch die Kopplungsglieder 96 in Ergänzung zu dem von der Unterseite 128 abstehenden, konusförmigen Kopplungsglied 99 eine Bewegung des Verbindungselements 14i relativ zur Knochenschraube 16 parallel zur Längsachse 72 verhindern.

Auch das Kopplungsglied 92 des Verbindungselements 14i definiert eine Vorzugsrichtung 116, die quer zur Längsachse 72 verläuft.

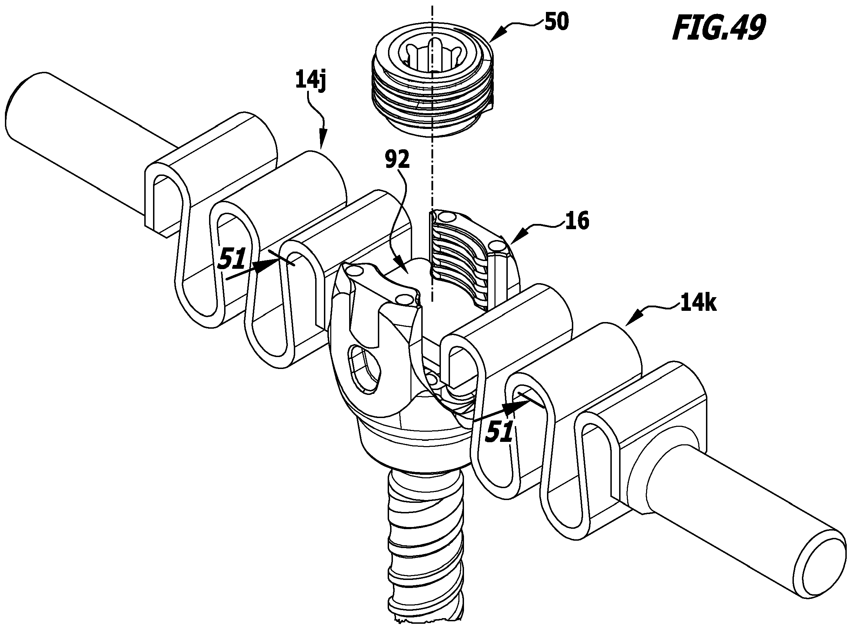

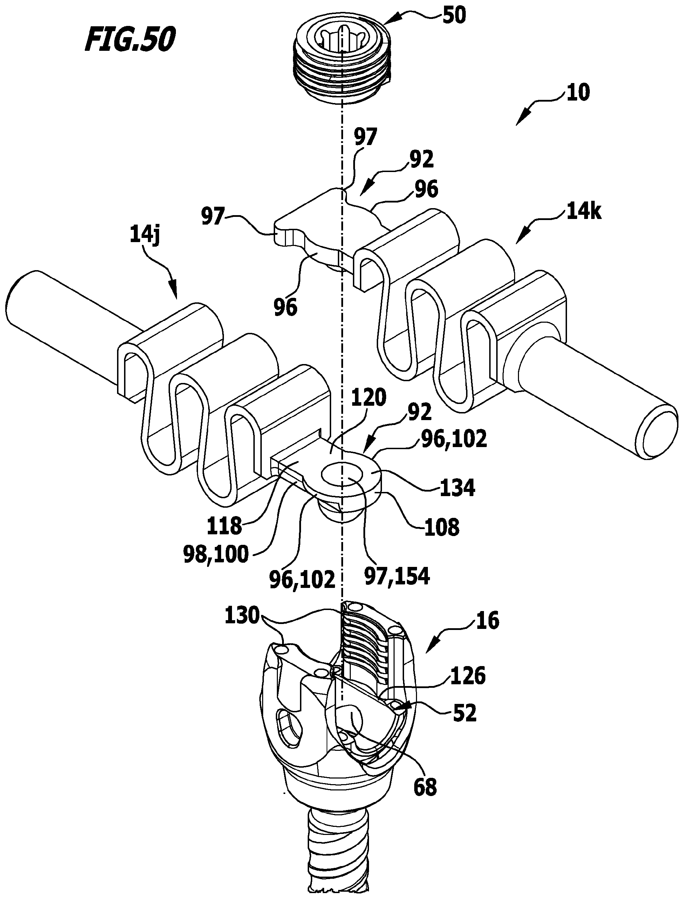

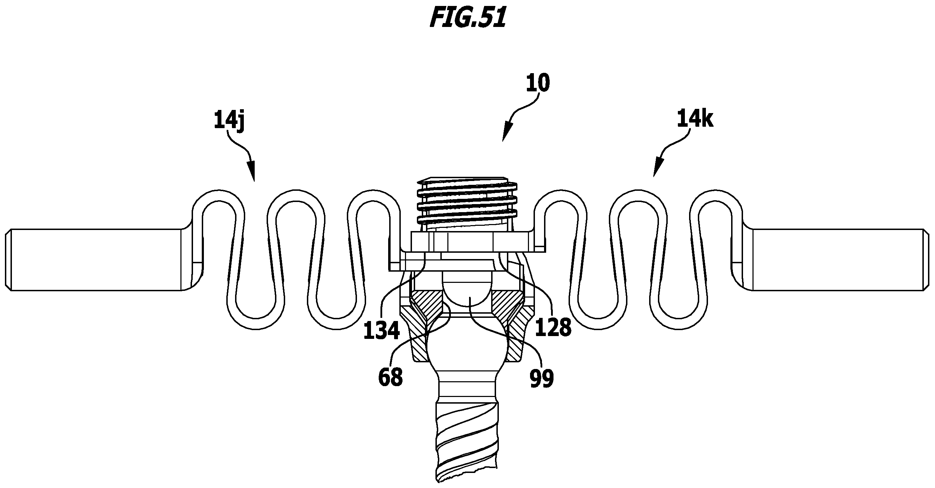

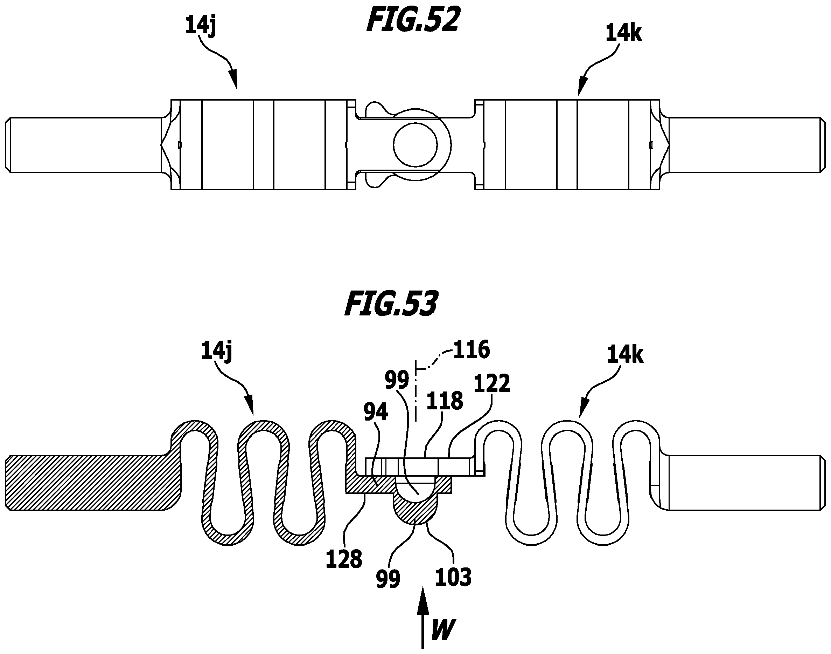

In den Figuren 49 bis 53 sind zwei weitere Ausführungsbeispiele von Verbindungselementen schematisch dargestellt und insgesamt mit den Bezugszeichen 14j beziehungsweise 14k bezeichnet. Sie unterscheiden sich von dem Verbindungselement 14 durch die Ausgestaltung ihrer Kopplungselemente 92.

Das Kopplungselement 92 des Verbindungselements 14j ist im Wesentlichen quaderförmig ausgebildet und weist ähnlich wie das Kopplungselement 92 des Verbindungselements 14a zwei in diametraler Richtung voneinander weisend vom Kopplungselementgrundkörper 94 abstehende Kopplungsglieder 96 auf in Form von Kopplungsvorsprüngen 102.

Ein vom Zwischenabschnitt 74 weg weisendes Ende des Kopplungselements 92 ist in Form einer zylindrischen Endfläche 108 geformt, so dass insgesamt eine flache zylindrische Scheibe ausgebildet wird. Die Oberseite 128 des Kopplungselements 92 bildet einen ebenen Flächenabschnitt 120. Von der Unterseite 128 steht quer zur Längsachse 72 ein Kopplungsglied 99 in Form eines im Wesentlichen zylindrischen Kopplungsvorsprungs 103 ab, dessen vom Kopplungselementgrundkörper 94 weg weisendes Ende kugelförmig abgerundet ist. Ein Außendurchmesser des Kopplungsglieds 99 ist an den Innendurchmesser der Bohrung 68 des Einsatzes 54 angepasst.

Ferner sind am Kopplungselement 92 zwei parallel zueinander verlaufende Kopplungsglieder 98 in Form von Rücksprüngen 100 ausgebildet, die sich bis zur Endfläche 108 erstrecken. Die Rücksprünge 100 sind so bemessen, dass sie auf den Kanten 126 des Einsatzes 54 aufliegen können.

Auf der Oberseite 118 des Kopplungselements 92 ist ein Kopplungsglied 97 in Form einer Kopplungsausnehmung 154 ausgebildet. Die Kopplungsausnehmung 154 ist hohlkugelig geformt. Sie ist im Wesentlichen koaxial zum Kopplungsglied 99 angeordnet und ausgebildet.

Die Oberseite 118 bildet ferner eine Verbindungselementanlagefläche 134. An diese ist eine korrespondierende Verbindungselementanlagefläche 134 des Kopplungselements 92 des Verbindungselements 14k anlegbar.

Das Kopplungselement 92 des Verbindungselements 14k weist eine ebene Oberseite 118 auf, die einen ebenen Klemmflächenabschnitt 122 für das Ende 124 der Fixierschraube 50 bildet. Der Kopplungselementgrundkörper 94 ist im Wesentlichen quaderförmig ausgebildet und weist ebenfalls zwei quer zur Längsachse 72 vorstehende Kopplungsglieder 96 auf. Diese definieren einen gemeinsamen Außendurchmesser, der einem Innendurchmesser des Innengewindes 46 entspricht.

Vom Zwischenabschnitt 74 weg weisend schließen sich an die Kopplungsglieder 96 Kopplungsglieder 97 in Form von Kopplungsvorsprüngen an, die eine zu den konkaven Seitenflächen 130 der Wandabschnitte 44 korrespondierende Form aufweisen.

Von der Unterseite 128 des Kopplungselements 92 des Verbindungselements 14k steht ein weiteres Kopplungsglied 99 ab, welches eine zur Kopplungsausnehmung 154 korrespondierende Form aufweist, so dass das Kopplungsglied 99 formschlüssig in die Kopplungsausnehmung 154 einführbar ist.

Die beiden Verbindungselemente 14j und 14k können mit ihren beiden Kopplungselementen 92 gemeinsam in einer Verbindungselementaufnahme 42 einer Knochenschraube 16 festgelegt werden. Zunächst wird das Kopplungselement 92 des Verbindungselements 14j in der beschriebenen Weise in die Verbindungselementaufnahme 42 eingelegt, so dass das Kopplungsglied 99 in die Bohrung 68 eintaucht. Nun kann das Verbindungselement 14k mit seinem Kopplungselement 92 in die Verbindungselementaufnahme 42 eingelegt werden, so dass das Kopplungsglied 92 in die Kopplungsausnehmung 154 eintaucht.

Mit der Fixierschraube 50 lassen sich die beiden Verbindungselemente 14j und 14k gemeinsam klemmend an der Knochenschraube 16 festlegen. Die besondere Ausgestaltung der Kopplungselemente 92 der Verbindungselemente 14j und 14k ermöglicht praktisch eine Verlängerung eines Verbindungselements auf einfache Weise.

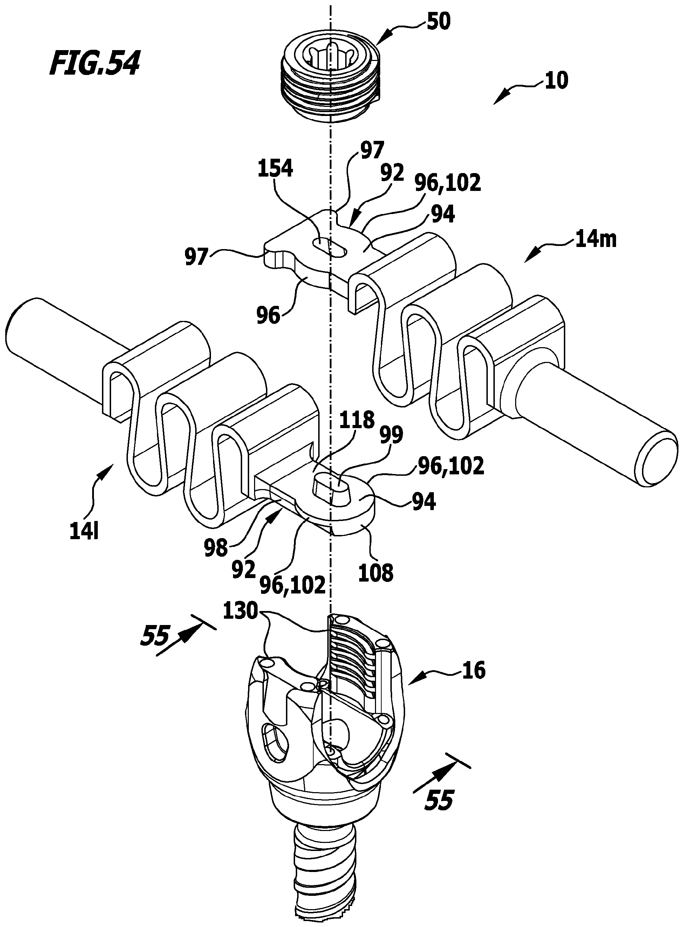

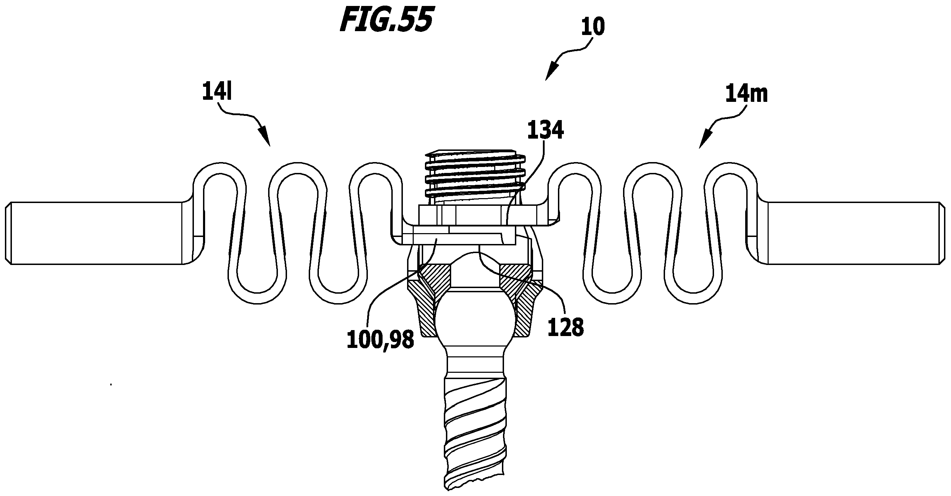

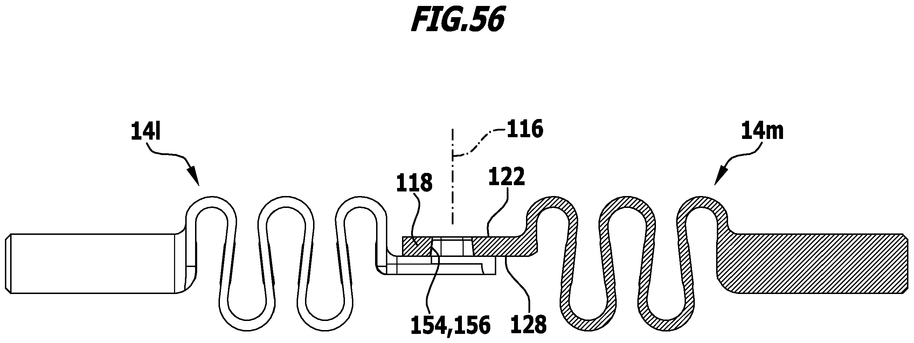

In den Figuren 54 bis 56 ist schematisch ein dreizehntes und ein vierzehntes Ausführungsbeispiel eines Verbindungselements dargestellt und mit den Bezugszeichen 14l und 14m bezeichnet. Die Verbindungselemente 14l und 14m unterscheiden sich vom Verbindungselement 14 durch die Ausgestaltung ihrer Kopplungselemente 92.

Das Kopplungselement 92 des Verbindungselements 14l weist einen im Wesentlichen quaderförmigen Grundkörper auf. Von diesem stehen quer zur Längsachse 72 und diametral voneinander weg weisend zwei Kopplungsglieder 96 in Form von Kopplungsvorsprüngen 102 ab. Diese sind konvex vom Kopplungselementgrundkörper 94 weg weisend gekrümmt und definieren gemeinsam einen Außendurchmesser, der einem Innendurchmesser des Innengewindes 46 entspricht.

Parallel zur Längsachse 72 sind an der Unterseite 128 zwei Kopplungsglieder 98 in Form von seitlichen Rücksprüngen 100 ausgebildet, die derart bemessen und angeordnet sind, dass sie auf die Kanten 126 des Einsatzes 54 aufgelegt werden können. Die Unterseite 128 ist eben. Die Oberseite 118 ist ebenfalls eben und bildet eine Verbindungselementanlagefläche 134 zum Anlegen an das Verbindungselement 14m. Ferner steht von der Oberseite 118 ein Kopplungsglied 99 in Form eines Kopplungsvorsprungs in einer Richtung quer zur Längsachse 72 ab. Das Kopplungsglied 99 ist im Wesentlichen langgestreckt quaderförmig ausgebildet mit abgerundeten Stirnkanten und schwach geneigten Seitenflächen, so dass insgesamt ein schwach konischer Vorsprung ausgebildet wird.

Das Kopplungselement 92 des Verbindungselements 14m weist einen im Wesentlichen quaderförmigen Kopplungselementgrundkörper 94 auf, von dem sich quer zur Längsachse 72 zwei Kopplungsglieder 96 in Form von Kopplungsvorsprüngen 102 in zueinander diametral entgegengesetzter Richtung weg erstrecken.

Die Kopplungsvorsprünge 102 sind vom Kopplungselementgrundkörper 94 weg weisend konvex gekrümmt ausgebildet und definieren gemeinsam einen Außendurchmesser, welcher einem Innendurchmesser des Innengewindes 46 entspricht.

An die Kopplungsglieder 96 schließen sich auf deren vom Zwischenabschnitt 74 weg weisenden Seite zwei Kopplungsglieder 97 in Form von Kopplungsvorsprüngen an, die eine Außenkontur aufweisen, welche an die Seitenflächen 130 der Wandabschnitte 44 angepasst ist.

Ferner ist das Kopplungselement 92 mit einer sich quer zur Längsachse 72 erstreckenden Kopplungsausnehmung 144 versehen, die in Form einer Durchbrechung 156 ausgebildet ist. Die Durchbrechung 156 weist im Wesentlichen die Form eines Langlochs mit abgerundeten Ecken auf. Ferner ist sie schwach konisch ausgebildet, so dass das Kopplungsglied 99 formschlüssig in die Kopplungsausnehmung 154 einsetzbar ist.

Die Unterseite 128 des Kopplungselements 92 des Verbindungselements 14m ist ebenso eben wie die Oberseite 118. Die Oberseite 118 bildet einen ebenen Klemmflächenabschnitt 122 für das distale Ende 124 der Fixierschraube 50.

Ähnlich wie die Verbindungselemente 14j und 14k können die Verbindungselemente 14l und 14m miteinander gekoppelt an einer einzigen Knochenschraube 16 gemeinsam festgelegt werden. Hierfür wird zunächst das Kopplungselement 92 des Verbindungselements 14l in die Verbindungselementaufnahme 42 eingelegt, so dass das Kopplungsglied 99 vom Befestigungsteil 24 weg weist. Danach kann das Kopplungselement 92 des Verbindungselements 14m in die Verbindungselementaufnahme 42 eingelegt werden, so dass die Kopplungsausnehmung 154 das Kopplungsglied 99 aufnimmt. Abschließend können die Kopplungselemente 92 klemmend am Halteteil festgelegt werden.

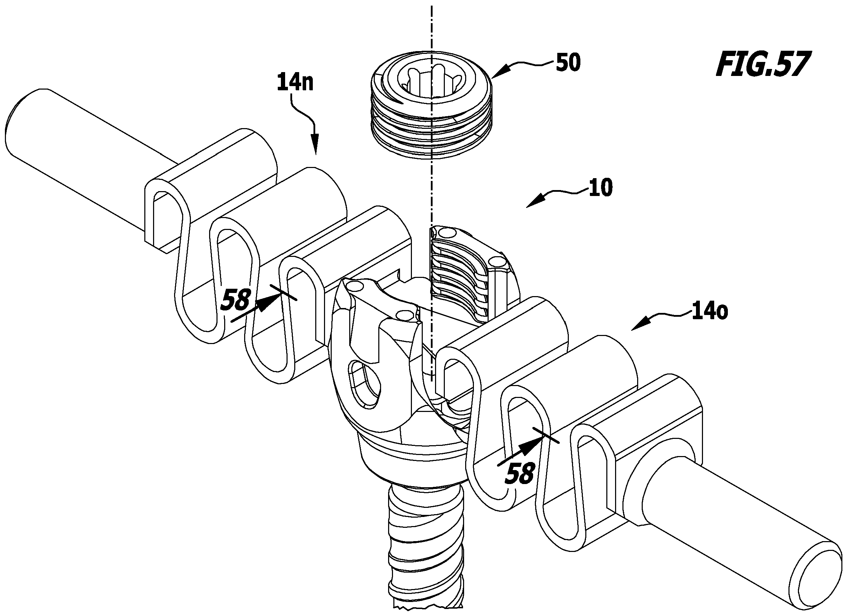

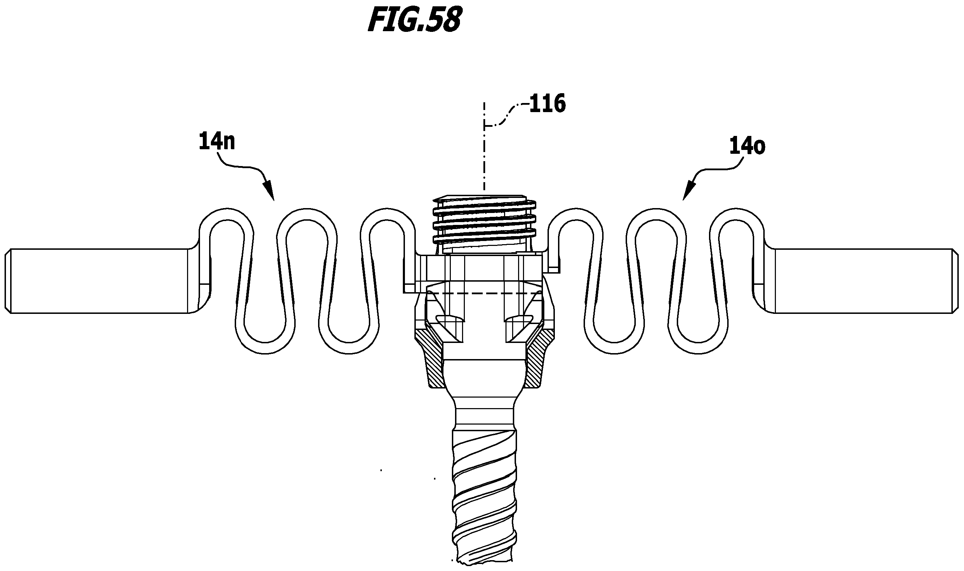

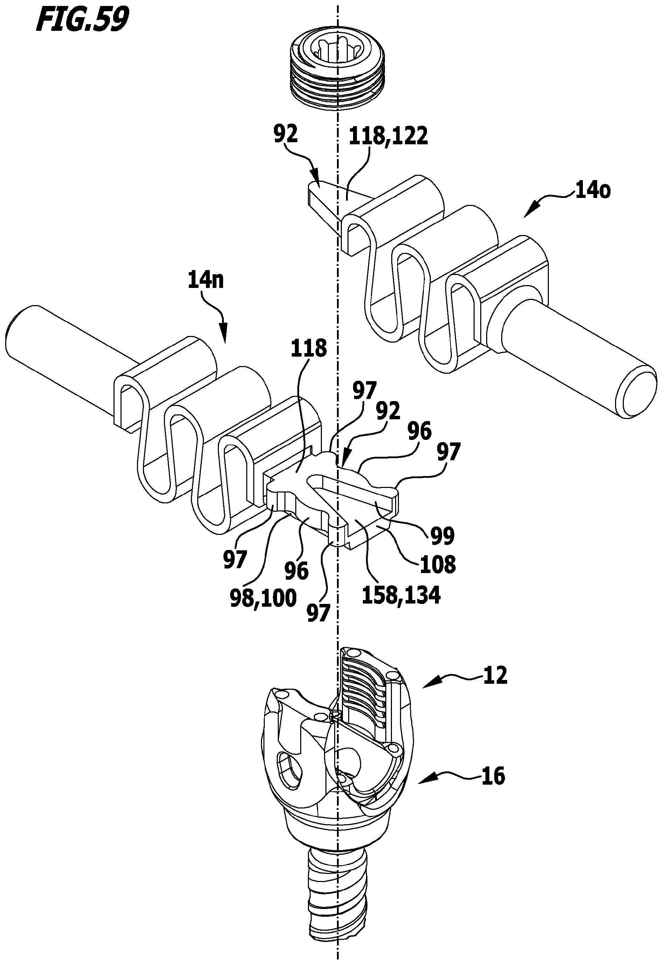

In den Figuren 57 bis 61 sind zwei weitere Ausführungsbeispiele von Verbindungselementen schematisch dargestellt und mit den Bezugszeichen 14n und 14o bezeichnet. Sie unterscheiden sich vom Verbindungselement 14 durch die Ausgestaltung ihrer Kopplungselemente 92.

Das Verbindungelement 14n weist ein Kopplungselement 92 auf, das im Wesentlichen dem Kopplungselement 92 des Verbindungselements 14h entspricht. Es unterscheidet sich lediglich durch die Ausbildung eines Kopplungsglieds 99 in Form einer Kopplungsausnehmung 154.

Die Kopplungsausnehmung 154 erstreckt sich ausgehend von der Endfläche 108 in Richtung auf den Zwischenabschnitt 74 hin, und zwar derart, dass ihre Breite abnimmt, sodass insgesamt ein gleichseitiges Prisma als Vertiefung ausgebildet wird. Die Kopplungsausnehmung 154 erstreckt sich zudem ausgehend von der Oberseite 118. Mit anderen Worten ist die Kopplungsausnehmung 154 von der Oberseite 118 und von der Endfläche 108 weg weisend geöffnet.

Die Oberseite 118 bildet ferner einen ebenen Klemmflächenabschnitt 122. Eine parallel zur Unterseite 128 verlaufende Begrenzungsfläche 158 der Kopplungsausnehmung 154 bildet eine Verbindungselementanlagefläche 134 zum Anlegen an das Verbindungselement 14o, nämlich dessen Kopplungselement 92.

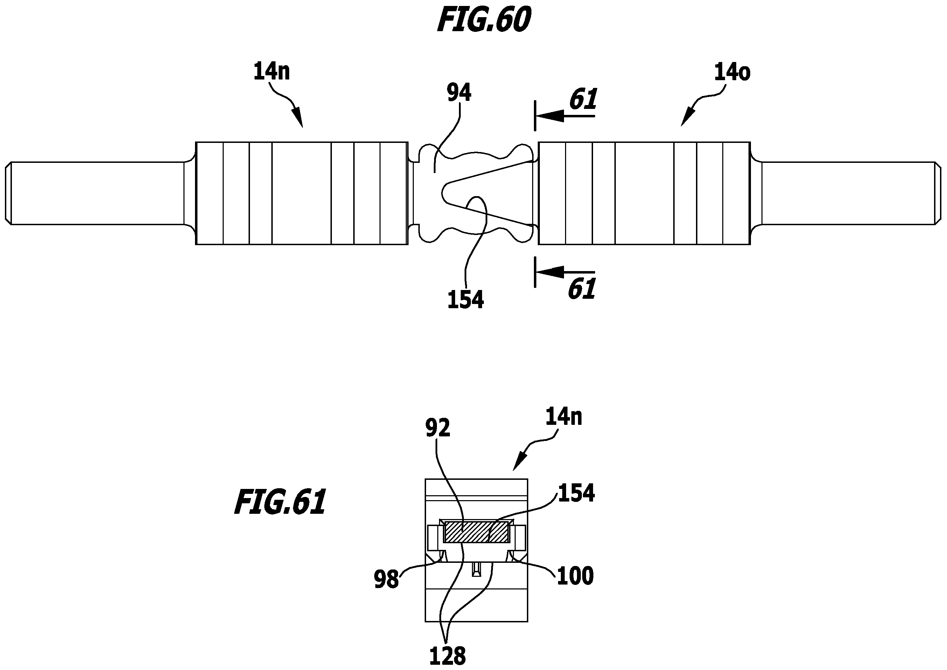

Das Kopplungselement 92 des Verbindungselements 14o weist die Form eines gleichseitigen Prismas auf und ist korrespondierend zur Kopplungsausnehmung 154 ausgebildet. Das Kopplungselement 92 kann somit vollständig in die Kopplungsausnehmung 154 eingeführt werden, wie dies beispielhaft in Figur 60 schematisch dargestellt ist.

Die Oberseite 118 des Kopplungselements 92 des Verbindungselements 14o bildet ferner einen ebenen Klemmflächenabschnitt 122. Die Unterseite 128 des Kopplungselements 92 liegt an der Begrenzungsfläche 158 an.

Die miteinander gekoppelten Kopplungselemente 92 der Verbindungselemente 14n und 14o bilden zusammen eine Einheit, die dieselbe Form aufweist wie das Kopplungselement 92 des Verbindungselements 14h. Mit der Fixierschraube 50 können die beiden Verbindungselemente 14n und 14o in einer Richtung parallel zur Längsachse 72 sowie gegen eine Rotation um dieselbe mit der Fixierschraube 50 an der Knochenschraube 16 gesichert werden.

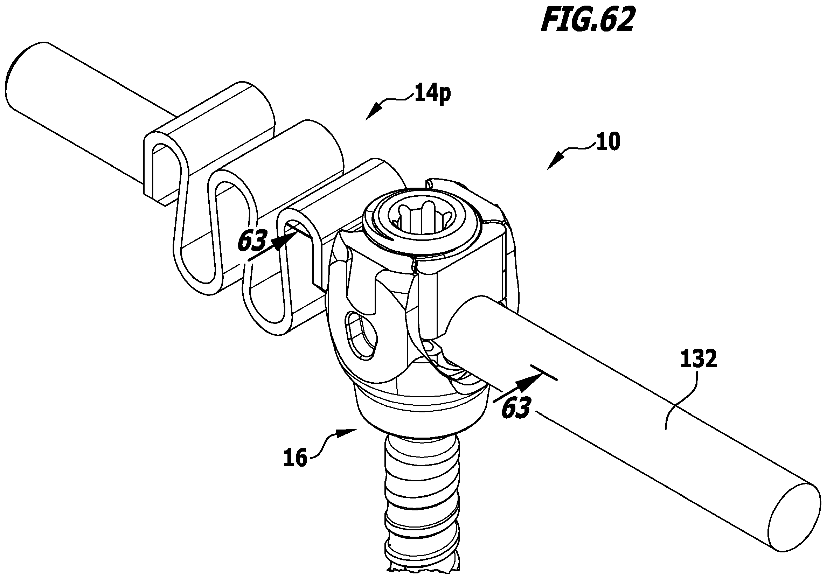

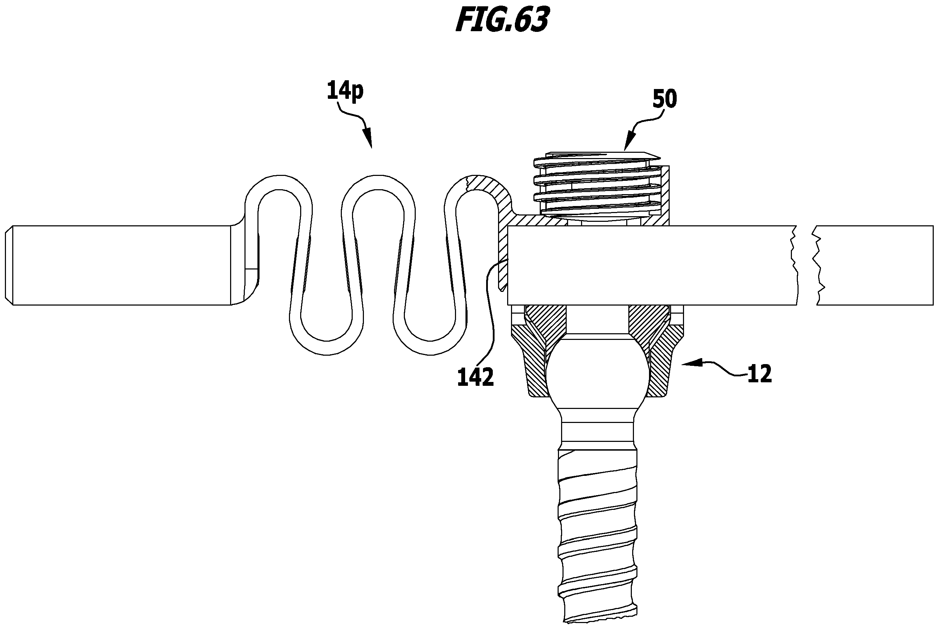

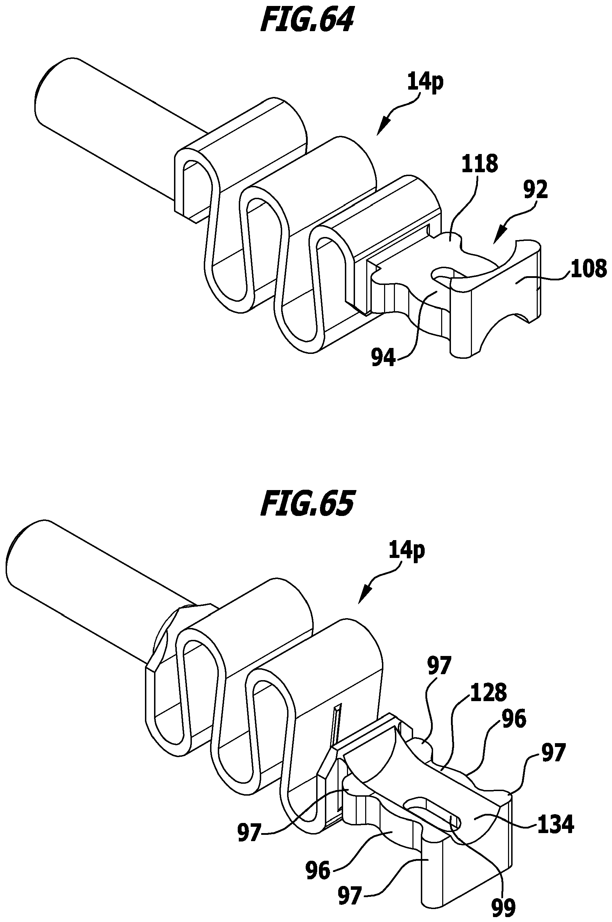

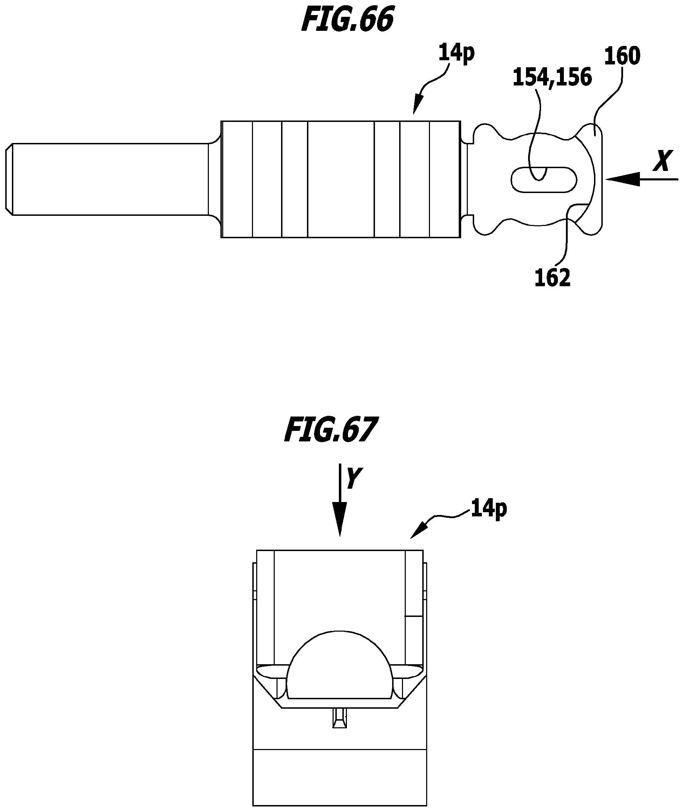

In den Figuren 62 bis 67 ist ein siebzehntes Ausführungsbeispiel eines Verbindungselements schematisch dargestellt und insgesamt mit dem Bezugszeichen 14p bezeichnet. Es unterscheidet sich vom Verbindungselement 14 durch die Ausgestaltung des Kopplungselements 92.

Das Kopplungselement 92 des Verbindungselements 14p entspricht in seinem Aufbau im Wesentlichen dem Kopplungselement 92 des Verbindungselements 14c. Von einem im Wesentlichen quaderförmigen Kupplungselementgrundkörper 94 stehen seitlich zwei Kopplungsglieder 96 in zueinander diametral gegengesetzten Richtungen weisend ab, die einen gemeinsamen Außendurchmesser definieren, welcher an einen Innendurchmesser des Innengewindes 46 angepasst ist.

Die beidseits der Kopplungsglieder 96 abstehenden Kopplungsglieder 97 weisen eine Außenkontur auf, die wiederum an die konkaven Seitenflächen 130 der Wandabschnitte 44 angepasst ist. Die am freien Ende des Kopplungselements 92 ausgebildeten Kopplungsglieder 97 erstrecken sich zudem über die Oberseite 118 hinaus und vergrößern damit die Endfläche 108 des Kopplungselements 92 im Vergleich zur Endfläche 108 des Kopplungselements 92 des Verbindungselements 14c. Insgesamt wird so eine Stirnwand 160 ausgebildet, die eine in Richtung auf den Zwischenabschnitt 74 weisende Seitenfläche 162 aufweist, welche in Form eines hohlzylindrischen Wandabschnitts ausgebildet ist. Ein von der Seitenfläche 162 definierter Innendurchmesser ist an einen Außendurchmesser des Außengewindes 48 der Fixierschraube 50 angepasst.

Eine Breite der Stirnwand 160 entspricht im Wesentlichen einem Abstand zwischen den beiden Wandabschnitten 44, so dass die Stirnwand 160 die Verbindungselementaufnahme 42 vom Zwischenabschnitt 44 weg weisend verschließt.

Das Kopplungselement 92 weit ferner ein weiteres Kopplungsglied 99 in Form einer Kopplungsausnehmung 154 auf, die als Durchbrechung 156 ausgebildet ist, und zwar in Form eines Langlochs. Die Durchbrechung verbindet die Oberseite 118 mit der hohlzylindrischen Verbindungselementanlagefläche 134, die auf einer Unterseite 128 des Kopplungselements 92 ausgebildet ist. Die Durchbrechung 156 ermöglicht die Verwendung einer Fixierschraube 50, die herkömmlicherweise eingesetzt wird, also kein im Wesentlichen ebenes distales Ende 124 aufweist, sondern ein spitz zulaufendes Ende.

Das Verbindungselement 14p ermöglicht es, analog dem Verbindungselement 14c, zusammen mit einem langgestreckten, kreiszylindrischen Verbindungsstab 132 in der Verbindungselementaufnahme 42 der Knochenschraube 16 mit der Fixierschraube 50 festgelegt zu werden. Mit dem Verbindungselement 14p lassen sich so Verbindungsstäbe 132 auf einfache Weise und ohne weitere Hilfsmittel verlängern.

Die oben beschriebenen Ausführungsbeispiele von Verbindungselementen können aus körperverträglichen Metallen oder Kunststoffen hergestellt sein. Vorzugsweise sind sie einstückig ausgebildet.

Die Kopplungselemente der Verbindungselemente lassen sich praktisch beliebig kombinieren. Sie können wahlweise an Verbindungselementen mit elastischen Zwischenabschnitten 74 angeordnet oder ausgebildet sein oder auch an inelastischen oder im Wesentlichen inelastischen Zwischenabschnitten, beispielsweise an kreiszylindrischen Verbindungsstäben. Insbesondere ist es auch denkbar, dass Verbindungselemente zwei Kopplungselemente aufweisen. Diese können identisch oder auch unterschiedlich ausgebildet sein.

Ferner lassen sich für die Kopplungselemente die beschriebenen Kopplungsglieder grundsätzlich beliebig miteinander kombinieren. Beispielsweise können an Kopplungselementen, die oben beschrieben wurden und keine Durchbrechungen aufweisen, auch Durchbrechungen vorgesehen sein.

## Patentansprüche

1. Verbindungselement (14c; 14p) für ein Wirbelsäulenstabilisierungssystem (10) mit einem ersten Ende (66) zum Festlegen an einer ersten Knochenbefestigungseinrichtung (12), einem zweiten Ende (70) zum Festlegen an einer zweiten Knochenbefestigungseinrichtung (12) und einem zwischen den beiden Enden (66, 70) angeordneten oder ausgebildeten, eine Längsachse (72) definierenden Zwischenabschnitt (74), wobei mindestens eines der beiden Enden (70) in Form eines Kopplungselements (92) ausgebildet ist zum Festlegen des Verbindungselements (14c; 14p) an einer Knochenbefestigungseinrichtung (12) in mindestens einer definierten Orientierung bezogen auf die Längsachse (72) und wobei das Kopplungselement (92) eine von einem Kreiszylinder abweichende Form und eine Verbindungselementanlagefläche (134) aufweist zum Anlegen an ein weiteres Verbindungselement (132), insbesondere an ein Ende des weiteren Verbindungselements (14; 14a; 14b; 14c; 14d; 14e; 14f; 14g; 14h; 14i; 14j; 14k; 14l; 14m; 14n, 14o; 14p; 132), wobei die Verbindungselementanlagefläche (134) mindestens teilweise in Form einer hohlzylindrischen Wandfläche (136) ausgebildet ist zum Aufnehmen eines in Form eines Kreiszylinders ausgebildeten und sich in Richtung der Längsachse oder parallel zu dieser erstreckenden Endes eines weiteren Verbindungselements (14; 14a; 14b; 14c; 14d; 14e; 14f; 14g; 14h; 14i; 14j; 14k; 14l; 14m; 14n, 14o; 14p; 132), wobei das Kopplungselement (92) einen Kopplungselementgrundkörper (94) und mindestens ein am Kopplungselementgrundkörper (94) angeordnetes oder ausgebildetes Kopplungsglied (96, 97, 99) umfasst, wobei sich das mindestens eine Kopplungsglied (96, 97, 99) in einer Kopplungsgliedrichtung (104) erstreckt, welche quer zur Längsachse (72) verläuft, wobei das mindestens eine Kopplungsglied (96, 97, 99) in Form eines Kopplungsvorsprungs (102) ausgebildet ist, **dadurch gekennzeichnet, dass** der Kopplungsvorsprung (102) ballig, kugelig, zylindrisch oder in Form einer Kombination einer oder mehrerer dieser Formen ausgebildet ist und dass der Kopplungselementgrundkörper (94) drei oder mehr Kopplungsglieder (96, 97, 99) umfasst.

2. Verbindungselement nach Anspruch 1, **dadurch gekennzeichnet, dass**
a) beide Enden (66, 70) in Form eines Kopplungselements (92) ausgebildet sind, welches eine von einem Kreiszylinder abweichende Form aufweist,
und/oder
b) das Kopplungselement (92) einstückig ausgebildet ist und/oder
c) das Verbindungselement (14c; 14p) einstückig ausgebildet ist.

3. Verbindungselement nach Anspruch 2, **dadurch gekennzeichnet, dass**
a) sich das mindestens eine Kopplungsglied in einer Kopplungsgliedrichtung erstreckt, welche parallel zur Längsachse (72) verläuft,
und/oder
b) der Kopplungselementgrundkörper (94) in Form eines geraden oder schiefen allgemeinen Zylinders ausgebildet ist und dass eine vom Zylinder definierte Zylinderlängsachse (116) quer zur Längsachse (72) verläuft
und/oder
c) das mindestens eine Kopplungsglied (96, 97, 99) in Form einer Kopplungsaufnahme (154) ausgebildet ist.

4. Verbindungselement nach Anspruch 3, **dadurch gekennzeichnet, dass** die Kopplungsaufnahme (154)
a) hohlballig, hohlkugelig, hohlzylindrisch, quaderförmig, in Form einer Durchbrechung (156) des Kopplungselementgrundkörpers (94) oder in Form einer Kombination einer oder mehrerer dieser Formen ausgebildet ist
und/oder
b) in einer Richtung quer und/oder parallel zur Längsachse (72) geöffnet ist.

5. Verbindungselement nach einem der voranstehenden Ansprüche, **dadurch gekennzeichnet, dass**
a) das Kopplungselement (92) bezogen auf eine die Längsachse (72) enthaltende Spiegelebene spiegelsymmetrisch ausgebildet ist und/oder dass ein Schwerpunkt des Kopplungselements (92) von der Längsachse (72) beabstandet ist
und/oder
b) eine vom Zwischenabschnitt (74) weg weisende Endfläche (108) des Kopplungselements (92) bezogen auf die Längsachse (72) geneigt oder abgerundet ist.

6. Verbindungselement nach einem der voranstehenden Ansprüche, **dadurch gekennzeichnet, dass** die Verbindungselementanlagefläche (134) mindestens teilweise in Form eines ebenen Anlageflächenabschnitts ausgebildet ist.

7. Verbindungselement nach einem der voranstehenden Ansprüche, **dadurch gekennzeichnet, dass** der Zwischenabschnitt (74) in Form eines streifenförmigen, gewundenen Blattfederelements (76) ausgebildet ist und mindestens eine in einer Ausnehmungsrichtung (82) quer zu einer vom Zwischenabschnitt (74) definierten Längsachse (72) seitlich geöffnete Ausnehmung (78, 80) aufweist.

8. Verbindungselement nach Anspruch 7, **dadurch gekennzeichnet, dass** das Kopplungselement (92) mindestens eine Vorzugsrichtung (116) quer zur Längsachse (72) definiert und dass die Vorzugsrichtung (116) und die Ausnehmungsrichtung (82) einen Orientierungswinkel einschließen, welcher in einem Bereich von 0° bis 180° liegt, vorzugsweise in einem Bereich von 0° bis 90°.

9. Verbindungselement nach einem der voranstehenden Ansprüche, **dadurch gekennzeichnet, dass** es aus einem metallischen Material oder aus einem Kunststoff hergestellt ist.

10. Verbindungselement nach einem der voranstehenden Ansprüche, **dadurch gekennzeichnet, dass** der Zwischenabschnitt mindestens teilweise flexibel ausgebildet ist.

11. Wirbelsäulenstabilisierungssystem (10) umfassend mindestens eine erste Knochenbefestigungseinrichtung (12), mindestens eine zweite Knochenbefestigungseinrichtung (12) und mindestens ein Verbindungselement (14c; 14p), **dadurch gekennzeichnet, dass** das mindestens eine Verbindungselement (14c; 14p) in Form eines Verbindungselements (14c; 14p) nach einem der voranstehenden Ansprüche ausgebildet ist.

12. Wirbelsäulenstabilisierungssystem nach Anspruch 11, **dadurch gekennzeichnet, dass** die mindestens eine erste und/oder die mindestens eine zweite Knochenbefestigungseinrichtung (12) in Form von Pedikelschrauben (16) ausgebildet sind, insbesondere in Form polyaxialer Pedikelschrauben (16).

13. Wirbelsäulenstabilisierungssystem nach Anspruch 11 oder 12, **dadurch gekennzeichnet, dass** die mindestens eine erste und/oder die mindestens eine zweite Knochenbefestigungseinrichtung (12) einen Befestigungsteil (24) und einen Halteteil (26) umfassen und dass der Halteteil (26) eine Verbindungselementaufnahme (42) zum Aufnehmen mindestens eines Endes (66, 70) eines Verbindungselements (14c; 14p) aufweist.

14. Wirbelsäulenstabilisierungssystem nach einem der Ansprüche 11 bis 13, **gekennzeichnet durch** zwei Verbindungselemente (14c; 14p), die zueinander korrespondierende Kopplungselemente (92) aufweisen, welche gemeinsam in einer Verbindungselementaufnahme (42) der mindestens einen ersten und/oder der mindestens einen zweiten Knochenbefestigungseinrichtung (12) festlegbar sind.

15. Wirbelsäulenstabilisierungssystem nach Anspruch 14, **dadurch gekennzeichnet, dass** die Kopplungselemente (92) der beiden Verbindungselemente (14c; 14p) zueinander korrespondierende Kopplungsglieder (96, 97, 99) aufweisen, die in einer Kopplungsstellung, in welcher die beiden Kopplungselemente (92) gemeinsam in einer Verbindungselementaufnahme (42) der mindestens einen ersten und/oder der mindestens einen zweiten Knochenbefestigungseinrichtung (12) gehalten sind, kraft- und/oder formschlüssig zusammenwirken oder miteinander in Eingriff stehen.

## Claims

1. Connection element (14c; 14p) for a spine stabilisation system (10) with a first end (66) for fixing to a first bone fixation device (12), a second end (70) for fixing to a second bone fixation device (12) and an intermediate portion (74) arranged or formed between the two ends (66, 70) and defining a longitudinal axis (72), wherein at least one of the two ends (70) takes the form of a coupling element (92) for fixing the connection element (14c; 14p) to a bone fixation device (12) in at least one defined orientation relative to the longitudinal axis (72) and wherein the coupling element (92) has a shape other than a circular cylinder and has a connection element contact face (134) for application against a further connection element (132), in particular against an end of the further connection element (14; 14a; 14b; 14c; 14d; 14e; 14f; 14g; 14h; 14i; 14j; 14k; 141; 14m; 14n, 14o; 14p; 132), wherein the connection element contact face (134) at least in part takes the form of a hollow-cylindrical wall face (136) for receiving an end of a further connection element (14; 14a; 14b; 14c; 14d; 14e; 14f; 14g; 14h; 14i; 14j; 14k; 141; 14m; 14n, 14o; 14p; 132) in the form of a circular cylinder and extending in the direction of the longitudinal axis or parallel thereto, wherein the coupling element (92) comprises a coupling element main body (94) and at least one coupling member (96, 97, 99) arranged or formed on the coupling element main body (94). wherein the at least one coupling member (96, 97, 99) extends in a coupling member direction (104) which extends transversely of the longitudinal axis (72), wherein the at least one coupling member (96, 97, 99) takes the form of a coupling projection (102), **characterized in that** the coupling projection (102) is crowned, spherical, cylindrical or takes the form of a combination of one or more of these shapes and **in that** the coupling element main body (94) has three or more coupling members (96, 97, 99).

2. Connection element according to claim 1, **characterized in that**
a) the two ends (66, 70) take the form of a coupling element (92) having a shape other than a circular cylinder,
and/or
b) the coupling element (92) is of one-piece construction
and/or
c) the connection element (14c; 14p) is of one-piece construction.

3. Connection element according to claim 2, **characterized in that**
a) the at least one coupling member extends in a coupling member direction which extends transversely of the longitudinal axis (72)
and/or
b) the coupling element main body (94) takes the form of a straight or oblique general cylinder and **in that** a cylinder longitudinal axis (116) defined by the cylinder extends transversely of the longitudinal axis (72)
and/or
c) the at least one coupling member (96, 97, 99) takes the form of a coupling receptacle (154).

4. Connection element according to claim 3, **characterized in that** the coupling receptacle (154)
a) is hollow-crowned, hollow-spherical, hollow-cylindrical, cuboidal, in the form of a through opening (156) in the coupling element main body (94) or in the form of a combination of one or more of these shapes
and/or
b) opens in a direction transverse and/or parallel to the longitudinal axis (72).

5. Connection element according to any one of the preceding claims,
**characterized in that**
a) the coupling element (92) is mirror-symmetrical relative to a mirror plane containing the longitudinal axis (72) and/or **in that** a centre of gravity of the coupling element (92) is spaced from the longitudinal axis (72)
and/or
b) an end face (108) of the coupling element (92) facing away from the intermediate portion (74) is inclined relative to the longitudinal axis (72) or rounded.

6. Connection element according to any one of the preceding claims, **characterized in that** the connection element contact face (134) at least in part takes the form of a planar contact face portion.

7. Connection element according to any one of the preceding claims, **characterized in that** the intermediate portion (74) is a winding leaf spring element (76) in the form of a strip and comprises at least one recess (78, 80) open laterally in a recess direction (82) transverse to a longitudinal axis (72) defined by the intermediate portion (74).

8. Connection element according to claim 7, **characterized in that** the coupling element (92) defines at least one preferential direction (116) which extends transversely of the longitudinal axis (72) and **in that** the preferential direction (116) and the recess direction (82) form an orientation angle that lies in a range from 0° to 180°, preferably in a range from 0° to 90°.

9. Connection element according to any one of the preceding claims, **characterized in that** it is made from a metallic material or a plastics material.

10. Connection element according to any one of the preceding claims, **characterized in that** the intermediate portion is at least in part flexible.

11. Spine stabilisation system (10) comprising at least one first bone fixation device (12), at least one second bone fixation device (12) and at least one connection element (14c; 14p), **characterized in that** the at least one connection element (14c; 14p) takes the form of a connection element (14c; 14p) according to any one of the preceding claims.

12. Spine stabilisation system according to claim 11, **characterized in that** the at least one first and/or the at least one second bone fixation device (12) takes the form of pedicle screws (16), in particular the form of polyaxial pedicle screws (16).

13. Spine stabilisation system according to claim 11 or 12, **characterized in that** the at least one first and/or the at least one second bone fixation device (12) comprises a fixing part (24) and a holding part (26) and **in that** the holding part (26) has a connection element receptacle (42) for receiving at least one end (66, 70) of a connection element (14c; 14p).

14. Spine stabilisation system according to any one of claims 11 to 13, **characterized by** two connection elements (14c; 14p), which have mutually corresponding coupling elements (92) which are jointly fixable in a connection element receptacle (42) of the at least one first and/or of the at least one second bone fixation device (12).

15. Spine stabilisation system according to claim 14, **characterized in that** the coupling elements (92) of the two connection elements (14c; 14p) comprise mutually corresponding coupling members (96, 97, 99), which in a coupling position, in which the two coupling elements (92) are held jointly in a connection element receptacle (42) of the at least one first and/or of the at least one second bone fixation device (12), co-operate in force- and/or positively-locking manner or are in engagement with one another.

## Revendications

1. Elément de liaison (14c ; 14p) pour un système de stabilisation de colonne vertébrale (10), pourvu d'une première extrémité (66) à fixer à un premier dispositif de fixation osseuse (12), d'une deuxième extrémité (70) à fixer à un deuxième dispositif de fixation osseuse (12) et d'une partie intermédiaire (74) agencée ou formée entre les deux extrémités (66, 70) et définissant un axe longitudinal (72), dans lequel au moins une des deux extrémités (70) est réalisée sous la forme d'un élément d'accouplement (92) pour fixer l'élément de liaison (14c ; 14p) à un dispositif de fixation osseuse (12) dans au moins une orientation définie par rapport à l'axe longitudinal (72) et dans lequel l'élément d'accouplement (92) présente une forme différente d'un cylindre circulaire et une surface d'appui d'élément de liaison (134) destinée à s'appliquer contre un autre élément de liaison (132), en particulier contre une extrémité de l'autre élément de liaison (14 ; 14a ; 14b ; 14c ; 14d ; 14e ; 14f ; 14g ; 14h ; 14i ; 14j ; 14k ; 14l ; 14m ; 14n ; 14o ; 14p ; 132), dans lequel la surface d'appui d'élément de liaison (134) est réalisée au moins en partie sous la forme d'une surface de paroi (136) cylindrique creuse pour loger une extrémité d'un autre élément de liaison (14 ; 14a ; 14b ; 14c ; 14d ; 14e ; 14f ; 14g ; 14h ; 14i ; 14j ; 14k ; 14l ; 14m ; 14n ; 14o ; 14p ; 132), ladite extrémité étant réalisée sous la forme d'un cylindre circulaire et s'étendant en direction de l'axe longitudinal ou parallèlement à celui-ci, dans lequel l'élément d'accouplement (92) comporte un corps de base d'élément d'accouplement (94) et au moins un organe d'accouplement (96, 97, 99) agencé ou formé sur le corps de base d'élément d'accouplement (94), dans lequel le au moins un organe d'accouplement (96, 97, 99) s'étend dans une direction d'organe d'accouplement (104), laquelle s'étend transversalement à l'axe longitudinal (72), dans lequel le au moins un organe d'accouplement (96, 97, 99) est réalisé sous la forme d'une partie saillante d'accouplement (102), **caractérisé en ce que** la partie saillante d'accouplement (102) est bombée, sphérique, cylindrique ou est réalisée sous la forme d'une combinaison d'une ou de plusieurs de ces formes et **en ce que** le corps de base d'élément d'accouplement (94) comporte trois organes d'accouplement (96, 97, 99) ou plus.

2. Elément de liaison selon la revendication 1, **caractérisé en ce que**
a) les deux extrémités (66, 70) sont réalisées sous la forme d'un élément d'accouplement (92), lequel présente une forme différente d'un cylindre circulaire,
et/ou
b) l'élément d'accouplement (92) est réalisé d'une seule pièce
et/ou
c) l'élément de liaison (14c ; 14p) est réalisé d'une seule pièce.

3. Elément de liaison selon la revendication 2, **caractérisé en ce que**
a) le au moins un organe d'accouplement s'étend dans une direction d'organe d'accouplement, laquelle s'étend parallèlement à l'axe longitudinal (72)
et/ou
b) le corps de base d'élément d'accouplement (94) est réalisé sous la forme d'un cylindre général droit ou oblique et **en ce qu'**un axe longitudinal de cylindre (116) défini par le cylindre s'étend transversalement à l'axe longitudinal (72)
et/ou
c) le au moins un organe d'accouplement (96, 97, 99) est réalisé sous la forme d'un logement d'accouplement (154).

4. Elément de liaison selon la revendication 3, **caractérisé en ce que** le logement d'accouplement (154)
a) est bombé creux, sphérique creux, cylindrique creux, parallélépipédique, réalisé sous la forme d'un orifice de passage (156) du corps de base d'élément d'accouplement (94) ou sous la forme d'une combinaison d'une ou de plusieurs de ces formes
et/ou
b) est ouvert dans une direction transversale et/ou parallèle à l'axe longitudinal (72).

5. Elément de liaison selon l'une quelconque des revendications précédentes, **caractérisé en ce que**
a) l'élément d'accouplement (92) est en symétrie spéculaire par rapport à un plan de symétrie contenant l'axe longitudinal (72) et/ou **en ce qu'**un centre de gravité de l'élément d'accouplement (92) est distant de l'axe longitudinal (72)
et/ou
b) une surface d'extrémité (108) de l'élément d'accouplement (92) orientée dans la direction opposée à la partie intermédiaire (74) est inclinée ou arrondie par rapport à l'axe longitudinal (72).

6. Elément de liaison selon l'une quelconque des revendications précédentes, **caractérisé en ce que** la surface d'appui d'élément de liaison (134) est réalisée au moins en partie sous la forme d'une partie de surface d'appui plane.

7. Elément de liaison selon l'une quelconque des revendications précédentes, **caractérisé en ce que** la partie intermédiaire (74) est réalisée sous la forme d'un élément ressort à lames (76) torsadé, en forme de bande, et présente au moins un évidement (78, 80) ouvert latéralement dans une direction d'évidement (82) transversale à un axe longitudinal (72) défini par la partie intermédiaire (74).

8. Elément de liaison selon la revendication 7, **caractérisé en ce que** l'élément d'accouplement (92) définit au moins une direction préférentielle (116) transversale à l'axe longitudinal (72) et **en ce que** la direction préférentielle (116) et la direction d'évidement (82) forment un angle d'orientation, lequel se situe dans une plage de 0° à 180°, de préférence dans une plage de 0° à 90°.

9. Elément de liaison selon l'une quelconque des revendications précédentes, **caractérisé en ce qu'**il est fabriqué à partir d'un matériau métallique ou à partir d'une matière plastique.

10. Elément de liaison selon l'une quelconque des revendications précédentes, **caractérisé en ce que** la partie intermédiaire est au moins en partie flexible.

11. Système de stabilisation de colonne vertébrale (10) comportant au moins un premier dispositif de fixation osseuse (12), au moins un deuxième dispositif de fixation osseuse (12) et au moins un élément de liaison (14c ; 14p), **caractérisé en ce que** le au moins un élément de liaison (14c ; 14p) est réalisé sous la forme d'un élément de liaison (14c ; 14p) selon l'une quelconque des revendications précédentes.

12. Système de stabilisation de colonne vertébrale selon la revendication 11, **caractérisé en ce que** le au moins un premier et/ou le au moins un deuxième dispositif de fixation osseuse (12) sont réalisés sous la forme de vis pédiculaires (16), en particulier sous la forme de vis pédiculaires polyaxiales (16).

13. Système de stabilisation de colonne vertébrale selon la revendication 11 ou 12, **caractérisé en ce que** le au moins un premier et/ou le au moins un deuxième dispositif de fixation osseuse (12) comportent une pièce de fixation (24) et une pièce de retenue (26) et **en ce que** la pièce de retenue (26) présente un logement d'élément de liaison (42) destiné à loger au moins une extrémité (66, 70) d'un élément de liaison (14c ; 14p).

14. Système de stabilisation de colonne vertébrale selon l'une quelconque des revendications 11 à 13, **caractérisé par** deux éléments de liaison (14c ; 14p), qui présentent des éléments d'accouplement (92) correspondant l'un à l'autre, lesquels peuvent être fixés conjointement dans un logement d'élément de liaison (42) du au moins un premier et/ou du au moins un deuxième dispositif de fixation osseuse (12).

15. Système de stabilisation de colonne vertébrale selon la revendication 14, **caractérisé en ce que** les éléments d'accouplement (92) des deux éléments de liaison (14c ; 14p) présentent des organes d'accouplement (96, 97, 99) correspondant les uns aux autres, qui coopèrent ou sont en contact l'un avec l'autre à force et/ou par coopération de formes dans une position d'accouplement dans laquelle les deux éléments d'accouplement (92) sont retenus conjointement dans un logement d'élément de liaison (42) du au moins un premier et/ou du au moins un deuxième dispositif de fixation osseuse (12).
